# EUROPEAN PATENT SPECIFICATION

(11) **EP 1 569 692 B1**
(45) Date of publication and mention of the grant of the patent: **10.11.2010**
(21) Application number: 02779843.8
(22) Date of filing: 25.11.2002
(51) Int. Cl.: A61K 45/06, A61K 31/616, A61K 31/205, A61P 9/00, A61P 1/00, A61P 3/10, A61P 7/02, A61P 35/00, A61P 29/00

(54) **Betaine and salicylic acid compositions**
Betain und Salicylsäure Zusammensetzungen
Compositions de bétaïne et d'acide salicylique

(43) Date of publication of application: 07.09.2005
(73) Proprietor: Messadek, Jallal, 4000 Liège (BE); Ennamany, Rachid, 33140 Villenave d'Ornon (FR)
(72) Inventor: MESSADEK, Jallal, B-4000 Liège (BE); ENNAMANY, Rachid, F-33800 Bordeaux (FR); THIRY, Michel, B-4870 Trooz (BE)
(86) International application number: PCT/IB2002/004923
(87) International publication number: WO 2004/049095

(56) References cited:
- WO-A-00/51596
- WO-A-97/06795
- WO-A-98/56497
- WO-A-99/45913
- WO-A-02/066002
- BE-A- 1 012 546
- BE-A- 1 012 712
- FR-A- 2 403 799
- FR-M- 2 590
- US-A- 4 066 756
- US-A- 4 703 045
- US-A- 5 961 999
- GURFINKEL ET AL: "Fast Platelet suppressionby lycine acetylsalicylate in chronic stable coronary patients, potential clinical impact over regular aspirin for coronary syndromes" JOURNAL OF THE AMERICAN COLLEGE OF CARDIOLOGY, vol. 35, no. 2Suppl.A, 2000, pages 408A-409A, XP008037129
- ZOELLEI I ET AL: "BETAINE-PALMITATE REDUCES ACETYLSALICYLIC ACID-INDUCED GASTRIC DAMAGE IN RATS" SCANDINAVIAN JOURNAL OF GASTROENTEROLOGY, XX, XX, vol. 36, no. 8, August 2001 (2001-08), pages 811-816, XP001027910 ISSN: 0036-5521

## Description

### FIELD OF THE INVENTION

This invention relates to the pharmaceutical combination comprising at least:
- A first compound selected among the group consisting of acetylsalicylic acid, salicylic acid, pharmaceutical salts thereof, and
- A second compound selected from the group consisting of glycine betaine anhydrous and glycine betaine monohydrate, with the provision that said second compound is different from the first compound and in an amount by weight at least 5 times the amount, calculated as acetylsalicyclic weight, of said first compound.

Further, the invention relates to pharmaceutical composition comprising a betaine and aspirin in a formulation wherein the betaine and aspirin are formulated together in a bilayered tablet, the aspirin being present in a first layer, and the betaine being present in a second layer in an amount at least five times the amount of aspirin. Said pharmaceutical composition wherein the tablet includes a core and a coating layer surrounding said core and wherein one of the betaine and aspirin is present in the core and the other is present in the coating layer surrounding the core.

### PRIOR ART

Glycine betaine is a molecule known for its osmo-protective properties, its cosmetic and pharmaceutical uses. WO 0051596 discloses the use of betaine for the treatment of thrombosis not induced by homocystinuria. In examples, said application discloses the combination of glycine betaine with a contrast agent.

Our recent studies point out the activity of Betaines or/and compounds of formula (CH₃)₃N⁺(CH₂)ₙCOO⁻ with n an integer from 1 to 5, preferably glycine betaine or a pharmaceutically acceptable salt thereof, esters thereof, precursors thereof, and mixtures thereof on P-selectin expression and to related diseases and pathologies induced by this glycoprotein. Consequently, therapeutic interventions directed against P-selectin or its ligand by compounds of formula (CH₃)₃N⁺(CH₂)ₙCOO⁻ with n an integer from 1 to 5 (preferably glycine betaine n=1), or a pharmaceutically acceptable salts thereof, esters thereof, precursors thereof, derivatives thereof and mixtures in the treatment thereof, may be beneficial in the treatment of thrombosis and in the treatment to related diseases and pathologies induced by this glycoprotein.

It is well know to administer to human doses of 1000 mg acetylsalicylic acid/day for treating pain relief, such as headaches, as well to administer doses of 100 mg to 500 mg acetylsalicylic acid /day as platelet anti aggregant for preventing thrombosis associated with atherosclerosis. These treatments are really effective, but have undesirable effects on patients subject to allergies or haemorrhage, especially when acetylsalicylic acid has to be administered every day, especially when acetylsalicylic acid has to be administered as platelet anti aggregant.

Despite its efficacy, antiaggregant treatment for preventing thrombosis with acetylsalicylic acid necessitates special precautions in use, such as overdose problems and unwanted side effects. This treatment makes it necessary to monitor patients, due in particular to haemorrhage-related problems which can arise during or after medication, gastro-intestinal mucosa damages, as well as possible incompatibility with other drugs.

PCT/BE 02/ 00013, of one of the applicants discloses a pharmaceutical combination comprising a therapeutic effective amount of a therapeutically active agent with at least one haemorrhagic side effect, and a therapeutic effective amount of a compound of formula (CH₃)₃N⁺(CH₂)ₙCOO⁻ with n an integer from 1 to 5, preferably glycine betaine or a pharmaceutically acceptable salt thereof, esters thereof, precursors thereof, and mixtures thereof for preventing or reducing said haemorrhagic side effect and/or for potentialising the therapeutic effect of said active agent. As possible active agent with haemorrhagic side effect, acetylsalicylic acid is given as example. The dosage forms given is the example 33 of said document comprise:
- acetylsalicylic acid 500 mg + 500 mg betaine + excipient
- acetylsalicylic acid 300 mg + 200 mg betaine + excipient, and
- acetylsalicylic acid 300 mg + 400 mg betaine + excipient.

US Patent 4,703,045 discloses a therapeutic composition containing betaine salts for the treatment of hangover, said oral composition comprising a pain relieving amount of analgesic (see claim 1 of said patent). In the example, unit doses according to compositions 4 and 12 of said patent comprises 200 mg acetylsalicylic acid for 2000 mg betaine citrate and other excipient, while unit dose of composition 15 comprises 110 mg acetylsalicylic acid for 2000 mg betaine and other excipient. The effervescent tablet of composition 19 comprises 250 mg of acetylsalicylic acid for 1750 mg betaine citrate and other excipient. Unit composition containing less than 100 mg acetylsalicylic acid does not contain a pain relief amount of analgesic for a human having a weight of about 70 kg.

US 4,703,045 describes the use of a combination of 2000 mg betaine citrate and 200 mg acetylsalicylic acid for treating hangover. Betaine citrate (C₁₁H₁₉NO₉ - CAS: 17671-50-0) has a molecular weight of 309.27. It is an equimolar association of a citrate molecule (MW 192.12) and betaine anhydrous molecule (MW 117.15). Hence, the correct amount of betaine in the combination is 2000 mg: 2.64 = 757.6 mg i.e. a ratio of 3.79: 1.

The publication "Betaine-Palmitate reduces acetylsalicylic acid-induced gastric damage in rats" (Zoelli et al, Scandinavian Journal Gastroenterology, vol. 36, no. 8, August 2001) describes the use of betaine-palmitate as a mucosal protective agent against acetylsalicylic acid challenge. Betaine-palmitate or palmitic acid prevented ASA-induced disruption of the mucosal barrier. However, betaine alone failed to have protective effect against ASA induced injuries in all tested conditions i.e. Histology, planimetry and ATP measurement and Vascular permeability index, as reported in the results chapter of the publication.

FR 2403799 describes injectable aqueous solutions comprising1000 weight parts of water, 20-100 weight parts of aspirin and 10-100 weight parts glutamic acid + betaine and also 105-150 weight parts of Arg or Lys. The ratio acetylsalicylic acid/betaine is 25:1.

US 4,066,756 describes bilayer tablets with aspirin in one layer and another active in the other layer. Aspirin is envisaged in low doses: 60-600 mg. However, the other active ingredient to reduce aspirin side effects is disodium cromoglycate. No mention or suggestion is made to glycine betaine.

WO 02/066002 describes bilayer tablets with aspirin in one layer and another active in the other layer. Aspirin is envisaged in low doses: 50-1000 mg. The other active, ranitidine, reduces the aspirin's side effects. No mention or suggestion is made to glycine betaine.

US Patent 6,235,311 discloses a pharmaceutical composition which is useful for cholesterol lowering and reducing the risk of a myocardial infarction, which includes a statin, such as pravastatin, lovastatin, simvastatin, atorvastatin, cerivastatin or fluvastatin, in combination with aspirin, in a manner to minimize interaction of aspirin with the statin and minimize side effects of aspirin. This pharmaceutical composition comprising a statin cholesterol lowering agent and aspirin in a formulation to reduce statin/aspirin interaction wherein the statin and aspirin are formulated together in a bilayered tablet, the aspirin being present in a first layer, and the statin being present in a second layer. The pharmaceutical composition as claimed in said patent is a the tablet including a core and a coating layer surrounding said core and wherein one of the statin and aspirin is present in the core and the other is present in the coating layer surrounding the core.

French Pat. No. 2590 M of 1963 describes the combination of betaine citrate with aspirin to buffer aspirin were the highest ratio of betaine citrate/aspirin is 5:3 i.e. 1,67 times the amount by weight of betaine citrate versus aspirin. The lowest amount of aspirin in each unitary dose is 300 mg.

French Pat. No. 1123 M of 1962 describes the synthesis of a betaine salicylate. Betaine salicylate is prepared through the interaction of betaine base and salicylic acid in anhydrous alcoholic medium with a ratio of 1.2: 1, i.e. 1,2 times the amount by weight of betaine versus aspirin

In this specification, "betaine" will refer to glycine betaine anhydrous or -monohydrate; and "aspirin" will refer to acetyl salicylic acid, salicycic acid, salts thereof, or mixtures thereof, both as described on page 1 herein, under Field of the Invention

None of these publications describe a pharmaceutical combination, advantageously as an unitary dose, wherein acetylsalicylic acid is less than 80 mg and Betaines is at least three times by weight the amount of acetylsalicylic acid, salicylic acid, pharmaceutical derivatives thereof and mixtures thereof.

None of these publications describe a pharmaceutical composition comprising a betaine and aspirin in a formulation wherein the betaine and aspirin are formulated together preferably in a bilayered tablet, the aspirin being present in a first layer, and the betaine being present in a second layer in an amount at least three times by weight the amount of aspirin.

None of these publications describe a pharmaceutical composition wherein the tablet includes a core and a coating layer surrounding said core and wherein one of the betaine and aspirin is present in the core and the other is present in the coating layer surrounding the core.

None of these publications describe a pharmaceutical composition wherein the tablet includes a core and a coating layer surrounding said core and wherein one of the betaine and aspirin is present in the core and the other is present in the coating layer surrounding the core and one or more of the Betaines and aspirin are formulated as a controlled release formulation.

None of these publications describe a pharmaceutical composition wherein the tablet includes a core constituted by aspirin and/or its pharmaceutically acceptable derivatives and a coating layer surrounding said core wherein one or more of the Betaines is present and formulated as a controlled release formulation.

None of these publications describe the therapeutically synergistic effect of betaine and aspirin allowing aspirin unitary dose amount reduction to less than 80 mg, while improving its therapeutic effect.

It has now been observed that it was possible to reduce the daily dose of acetylsalicylic acid for a human with a weight of 70 kg to less than 100 mg, when administrated in combination with a betaine while preventing thrombosis without special precautions in use, without overdose problems and without unwanted side effects. This treatment makes it possible to no more render necessary to monitor patients for haemorrhage-related problems which can arise during or after medication, as well as possible incompatibility with other drugs. Moreover, betaine prevents acetylsalicylic acid gastrointestinal induced mucosa damage when administrated in combination. Due to betaine antithrombotic properties it have appears that administrating the pharmaceutical combination of the invention allows to substantively reduce the amount of acetylsalicylic acid while achieving a significant therapeutic effect. In fact betaine and acetylsalicylic acid act in a synergistic manner with a good therapeutic effect in various pathologies such as blood flow disturbances, thrombo-embolism, inflammation and cancer.

The invention relates thus among others to:
Pharmaceutical combination as a unitary dose comprising at least:
   - A first compound selected among the group consisting acetylsalicylic acid, salicylic acid, pharmaceutical salts thereof, and mixtures thereof, and
   - A second compound selected from the group consisting of glycine betaine anhydrous and glycine betaine monohydrate with the provision that said second compound is different from the first compound
in which said combination comprises less than 80 mg of said first compound expressed as acetylsalicylic acid, and
in which the amount of second compound is at least five times the amount, calculated as acetylsalicylic acid weight, of said first compound.

### BRIEF DESCRIPTION OF THE INVENTION

The use of aspirin for reducing the risk of a myocardial infarction and the use of betaine for preventing or treating atherosclerosis and cardiovascular disease and cerebrovascular disease are well documented.

Aspirin is known for causing gastrointestinal bleeding when used for long-term therapy. It is therefore desirable in long-term aspirin therapy that the aspirin is provided in a form and an amount which minimizes side effects.

The aim of the present invention is to lower the aspirin amount needed to achieve a therapeutically effect when combining aspirin with betaine. Due to betaine antithrombotic properties the two drugs act in a synergistic manner for a powerful effect. Moreover betaine reduces aspirin induced side effects as haemorrhage and gastrointestinal damages. The combination of the invention may be useful for long term therapies as vascular occlusive diseases, inflammation, cancer and diabetes and aging related pathologies.

In view of the above, it is seen that there is a long-felt want in patients required to take both a betaine and aspirin for a betaine-aspirin formulation which provides for maximum reduction of risk of a myocardial infarction without the undesirable side effects and drug interaction normally associated with use of such combination.

Glycine betaine anhydrous or -monohydrate in the context of the present invention is used in combination with acetylsalicylic acid, salicylic acid, pharmaceutical salts thereof for various clinical applications, such as: coronary thromboses and venous thromboses
- thrombosis and re occlusion of the vascular system following a thrombolysis or an angioplasty
- ischemia reperfusion
- acute disseminated intravascular coagulation
- P-selectin related troubles
- infarct, angina pectoris, aneurysm, pulmonary embolism, phlebitis
- cerebral, thrombosis and thrombo-embolism
- post-traumatic shock, whether or not of surgical origin
- prevention of accidents of microcirculation in the following cases: haemophilia, chemotherapy, ageing, oral contraception using oestrogen's, obesity, tobacco addiction, prosthesis, claudication, diabetes.
- prevention of the risks associated with the administration of contrasting ionic and non ionic products.
- The extracorporeal circulation and the haemodialysis procedures. The blood in contact with artificial surfaces of patients subject to an extracorporal circulation has a risk of formation of platelet nails, of thrombi and embolism. These risks can be prevented by administering the compound(s) of the invention before and/or during and/or after these events.
- Reduction of atherosclerotic events (myocardial infarction, stroke, death due to vascular causes and restinosis) in patients with a history of symptomatic atherosclerotic disease defined by ischemic stroke, myocardial infarction or established peripheral arterial or venous disease.
- Inflammation phenomena. When binding it with integrine of Mac-1 receptor of the leukocytes and by reducing the expression of mitogenes and pro inflammation cytokines. When acting on the Mac-1 receptor, the compounds of the invention reduce the adhesive and migration properties of the leukocytes reducing thereby the tissue aggression.
- Stings and bites of venomous animals. Experimental data show that the injection of compounds of the invention to rats to which a venom lethal dose is injected, delays the death thereof. The compounds of the invention are therefore suitable for entering into antidote composition for venom, possibly in combination with other antivenomous compound(s).
- Prevention of blood circulation problems due to contact with artificial surfaces, such as biomaterial elements, prosthesis, etc. (balloons, catheters, hip prosthesis, stents, prosthetic cardiac valves, arterial gafts, etc.). When using these elements with the compounds of the invention, the secondary effects are reduced. Moreover coating these exougenous materials with the compounds of the invention avoid problems as reocclusion, rethrombosis and restinosis.
- Metastasis Prevention of cancerous cells. This anti tumoral activity is bound to the fact that cancerous cells released from tumours are transported by the micro thrombi inside the vascular system. These cancerous cells are undetectable by the immune system able to destroy them. Moreover, their incorporation in the micro thrombi facilitates their binding to the vascular system or in the organs, and creates then new cancerous colonies. As the formation of thrombi is function to the adhesion of fibrinogen to glycoprotein IIb IIIa site on the activated platelets, an antagonist of fibrinogen adhesion has an anti-metastasis activity by permitting the immune system to detect the cancerous cells during their migration, and by removing the vehicle (thrombus) enabling their transport and their binding. The compounds of the invention can be administered alone or with other anti-cancerous compounds (simultaneous administration or not) so as to improve their efficiency and the process of angiogenesis during malignant melanomas.
- Process for avoiding thrombo-embolic problems correlated to air trips. In view of its very low toxicity and its blood fluidifying characteristics, the compounds of the invention can be administered in the form of patch, sweets, confectioneries, cookies, drinks, meals, candies, etc. so as to prevent thromboembolic events for airplane /flight passengers.
- Sweetener for diabetes, the betaine being or not associated with another sweetener. As Betaine is a residue of sugar production, betaine has some sweetening properties which can be used for the preparation of sweetener with anti aggregation properties. Said sweetener, while avoiding circulation problems bound to diabetes, could improve the efficiency of insulins. It has been demonstrated that the activation of vitronectin receptors facilitates the cell migration and provides the necessary signals for the regulation and proliferation of cells, and potentialises the insulin effect (Ruoslahti, Kidney Int., 1997, 51, 1413-1417)
- As anti bacterial and anti infectious
- In combination with antibiotics
- In combination with insulin
- In combination with non steroidal anti inflammatory drugs
- Use of a combination of the invention for the treatment or for the prevention of troubles bound to one or more glycoprotein, especially to the receptor of one or more glycoprotein, preferably to the receptors of glycoprotein Ib and IIb IIIa.
- Use of a combination of the invention for potentializing the therapeutically effect of a pharmaceutical active agent.

### DESCRIPTION

The pharmaceutical compositions of the invention which includes a combination of a betaine and aspirin is effective in preventing, reducing and/or treating atherosclerosis, cardiovascular events and disease including coronary events and cerebrovascular events, and coronary artery disease and/or cerebrovascular disease, cancer, inflammation, diabetes and troubles related to aging.

The terms "cardiovascular event(s)" and "cardiovascular disease" as employed herein refer to coronary and/or cerebrovascular event(s) and disease including primary myocardial infarction, secondary myocardial infarction, myocardial ischemia, angina pectoris (including unstable angina), congestive heart failure, sudden cardiac death, cerebral infarction, cerebral thrombosis, cerebral ischemia, transient ischemic attack and the like.

The term "coronary artery disease" (CAD) as employed herein refers to diseases including atherosclerosis of the coronary arteries, previous myocardial infarction, ischemia, angina pectoris and/or heart failure.

The term "cerebrovascular disease" as employed herein refers to diseases including atherosclerosis of the intracranial and/or extracranial arteries, cerebral infarction, cerebral thrombosis, cerebral ischemia, stroke, and/or transient ischemic attacks.

"Lipidic betaines" and "betaine lipids", which are not part of the claimed invention, refer to betaine lipids which are structural components of membranes commonly found in ferns, mosses, fungi, amoeba, eukaryotes such as nonseed plants and algae. Betaine lipids are ether-linked, nonphosphorous glycerolipids that resemble the more commonly known phosphatidylcholine in overall structure. Most common glycerolipids are containing a diacyl-glycerol moiety to which a polar head group is attached. This head group can be a carbohydrate moiety as in the very abundant plant galactolipids or a phosphorylester as in the glycerophospholipids, the most common lipid class in animals. Betaine lipids represent a third class of glycerolipids in which a quaternary amine alcohol is bound in an ether linkage to the diacylglycerol moiety. They can be obtained by extraction, by biosynthesis or by synthesis. The betaine lipid diacylglyceryl- *O*-4' - (N, N, N- trimethyl)homoserine and a closely related isoform diacylglyceryl - *O*-2' - (hydroxymethyl) (N, N, N- trimethyl)-β-alanine are the most common.

Aspirin will preferably be employed in the form of salicylic acid acetate also referred to as acetylsalicylic acid.

In one embodiment salicylic acid may be employed.

### Background

Platelet aggregation is an essential event in the formation of blood clot and thrombus. In normal conditions, following a vascular lesion, blood clots prevent blood losses by closing the opening. However, in some pathological instances, the formation of a blood clot can reduce partly or completely the blood circulation, with the consequence of a cellular necrosis.

For example, the platelet aggregation and thus the thrombosis at the level of the atherosclerosis plaques is an important factor for the genesis of conditions such as angina pectoris, myocardium infarct and vessel occlusion following a thrombolysis or an angioplasty. Patients suffering a heart attack are treated with thrombolytic agents such as plasmin activators and the streptokinases which dissolve the fibrin from the clots. A major complication of this therapy is the reocclusion of vessels due to platelet aggregation, which can lead to irreversible damages to the heart, the brain or other organs.

Thrombosis starts with the adhesion of platelets at the vascular lesion sites. The platelet adhesion is initiated by the receptor located at the surface of the platelets which bind to proteins of the extracellular cellular matrix of the exposed endothelium, such as fibrinogen, fibronectin, Von Willebrand factor, as well as other adhesive proteins such as vibronectin, collagen and laminin. Therefor, the activation of platelets is a reply to agonists such as epinephrine, ADP, collagen, the arachidonic acid or the thrombin. This activation leads to the activation of the glycoprotein Ib receptor (GP Ib) and/or of the glycoprotein IIb IIIa receptor (GP IIb IIIa) at the surface of the platelets. This receptor(s) (GP Ib and/or GP IIb IIIa) is/are then available for its/their binding to fibrinogen and the platelet aggregation. The adhesion of the receptor (GP IIb IIIa) to other adhesive proteins such as the Von Willebrand factor also leads the attachment of platelets between them and their aggregation. The adhesion of molecules such as fibrinogen or the Von Willebrand factor to the receptor (GP IIb IIIa) leading the platelet aggregation is an essential step in the formation of the thrombus. The receptor (GP IIb IIIa ) is thus a privileged target for the new therapy treating thrombosis and thromboembolic pathologies. Furthermore, the use of antagonists of the glycoprotein IIb IIIa receptor inhibits the platelet aggregation, while respecting the other haemostasis mechanisms, is highly desirable in the new therapies bound to thrombosis. Several molecules having this antagonist property are marketed with usage restrictions due to immuno-reactivity problems, toxicity, allergy or hypersensibility reactions for some patients. A subject matter of the present invention is to propose a molecule, especially a well-known and used molecule of vegetal origin, having this antagonist activity for the glycoprotein IIb IIIa receptor, while not having toxic characteristics.

It is also known that the activation of the vitronectin receptor improves the cell migration and provides regulating signals of the cell proliferation and cell differentiation, and activates the effects of insulin (Ruoslahti, Kidney Int., 1997, 51, 1413-1417). The regulation of the vitronectin receptor is associated with pathological conditions, such as vascular restinosis (Clemetson and Clemetson, Cell.Mol. Life Sci., 1998,54,502-513), bone excess resorbtion (Rodan and Rodan, J. Endocrinol., 1997, 154 Suppl, S47-56), and the angiogenesis process during the malignant melanomas (Cheresh, Cancer Metastasis Rev., 1991, 10,3-10).

Surprisingly, it has now been found that betaines have an antagonist activity for one or more glycoprotein(s) receptors, such as the glycoprotein Ib receptor and the glycoprotein IIb IIIa receptor, by inhibiting the platelet aggregation induced by various agonists. This antagonist activity is not restricted to the glycoprotein site IIb IIIa but to all glycoprotein sites implicated in the cell adhesion of various origins, there between.

Surprisingly, it has now been found that betaines, when combined to acetylsalicylic acid act in a synergistic manner in different pathologies. This synergistic activity permit to lower the aspirin amount needed to achieve a therapeutically effect.

Platelets are activated by some agonists, whereby their forms, as well as their secretions of their granules can be modified, and whereby the aggregation thereof can be induced and the formation of clots and thrombi can be produced.

The present used platelet aggregation inhibitors are acting only on a single agonist. For example, aspirin is active against the arachidonic acid, ticlopidin is active against ADP, hirudin is active against thrombin. The Betaines of the invention disclosed here before are actives against various agonists, as well as on fibrinogen, fibronectin, Von Willebrand factor and other adhesive proteins such as P-selectin, vitronectin, collagen, laminin families and lectin families. This is a major improvement for their efficiency, while preserving the haemostasis mechanism so as to avoid haemorrahgic or bleeding events. Due to their activity by oral administration, said compounds are excellent candidates for pathologies with adhesion of cells between them.

In view of its very low toxicity and its efficiency, the best results have been obtained with glycine betaine.

None of the publications to which reference is made in the present specification teach the antagonist activity of the betaine/aspirin combination with respect to glycoprotein IIb IIIa receptor, nor its activity with respect to adhesive proteins. This antagonist activity is not only limited to the site of glycoprotein IIb IIIa, but also to all the other glycoproteic sites acting in the adhesion of cells of various origins there between.

In the present invention, anhydrous glycine betaine can be administered, or glycine betaine monohydrate.

Acetylsalicylic acid, salicylic acid and pharmaceutically acceptable salts are known to have platelet antiaggregant properties. However, the daily dose for ensuring a safe anti-aggregation for human with a weight of 70kg is at least 75 to 500 mg acetylsalicylic acid/ day. High daily dose causes high bleeding risks, whereby requiring monitoring patients, due in particular to haemorrhage-related problems which can arise during or after medication. With respect to low doses, it is also required to monitor the patients so as to check that the low doses are effective for ensuring the required platelet anti-aggregation. It is not possible for the moment to determine before the treatment is made whether the low dose of aspirin will be sufficient for a patient. For theses reasons, patients requiring an administration of aspirin as platelet antiaggregant receive daily doses of at least 125 mg.

It has now been discovered that it was possible to reduce the daily doses of aspirin and/or the requirement of monitoring the patient, while ensuring the prevention of arterial and venous thrombosis for substantially all types of patients in need of treatment and while avoiding substantially all bleeding risks, haemorrhage-related problems and aspirin induced gastro-intestinal damages which can arise during or after medication, as well as possible incompatibility with other drugs.

The invention relates thus to a pharmaceutical combination as a unitary dose comprising at least:
- A first compound selected among the group consisting acetylsalicylic acid, salicylic acid, mixtures thereof, and
- A second compound selected from the group consisting of betaine as defined previously, with the provision that said second compound is different from the first compound
in which said combination comprises less than 100 mg of said first compound expressed as acetylsalicylic acid, and
in which the amount of second compound is at least five times the amount, calculated as acetylsalicylic acid weight of said first compound.

The pharmaceutical combination is for example suitable for a four times a day administration or preferably for a daily administration. (advantageously a two times a day administration, preferably an once day administration).

### Oral formulations

The invention as claimed is limited to oral formulations.

Advantageously, the combination comprises an amount of said first compound, calculated as acetylsalicylic acid, of less than 80 mg, advantageously of less than 60 mg, preferably of less than 40 mg, even if said combination is administered as a daily administration in one or more doses.

For example, the combination comprises an amount of acetylsalicylic acid corresponding to 5 to 80 mg, advantageously from 10 to 75 mg, preferably from 15 to 75 mg calculated as acetylsalicylic acid. Specific examples of combination comprise 10, 15, 20, 25,30,40,50 and 70 mg of acetylsalicylic acid.

The amount of second compound is at least 5, advantageously at least 10 times the amount, calculated as acetylsalicylic acid weight, of said first compound, preferably at least 20 times the amount, calculated as acetylsalicylic acid, of said first compound. For example, the amount of second compound in the combination is 30, 40, 50, 70, 85, 100 times the amount, calculated as acetylsalicylic acid, of said first compound.

According to a specific embodiment, the combination is prepared at least from a mixture in which at least 50% by weight of the first compound and at least 50% of the second compound are in soluble form. Preferably, the combination is prepared at least from a mixture in which at least 90% by weight of the first compound and at least 90% of the second compound are in soluble form, most preferably from a mixture in which the first compound and the second compound are substantially completely in soluble form. This is advantageous for ensuring a homogeneous dispersion of the active compounds in the batch used for the preparation of the unit dose.

The combination of the invention is advantageously at least a controlled release combination for the second compound and/or at least an immediate release combination for the first compound. Preferably, the combination is a controlled release formulation for at least a part of the second compound and a substantially immediate release formulation for the first compound. The combination is for example a formulation enabling an immediate release for the first compound and for an amount of the second compound corresponding to two to ten times the amount of the first compound, and enabling a controlled release for an amount of second compound corresponding to more than 5, (preferably more than 10, most preferably more than 20) times the amount of first compound. The weight ratio amount of second compound in a controlled release form / amount of second compound in an immediate release form is for example comprised between 5:1 and 75:1, preferably between 10:1 and 50:1.

Possibly, the first compound can also be partly in a form suitable for a controlled release.

According to a specific embodiment, the combination is a formulation ensuring a first immediate release of an amount of second compound, then an immediate release of the first compound, and thereafter a controlled release of an larger amount of second compound.

The weight ratio amount of second compound in a controlled release form /amount of second compound in an immediate release form is for example comprised between 5:1 and 75:1, preferably between 10:1 and 50:1.

Immediate release form means in the present specification a form from which an active compound is released in the body so as to be bioavailable, less than 30 minutes after administration, advantageously less than 15 minutes after administration.

Controlled release form means in the present specification a form from which the release of an active compound is controlled during the time, such as delayed release and/or extended release. Advantageously, the controlled release form is a form suitable for ensuring a minimum active agent concentration in the blood during at least 4 hours, advantageously during at least 6 hours, preferably during at least 8 hours, most preferably during 12 hours or more than 12 hours , such as during 24 hours or more.

According to an embodiment, the combination comprises dry particles, especially micro particles, prepared by drying a mixture in which the first compound and the second compound are partly in a soluble form. In such dry particles, the first compound is homogeneously dispersed in the second compound, whereby enabling a simultaneously release of the first compound and of the second compound.

The first compound and the second compound can also be combined in the form of a matrix and or in the form of a mixture of dry particles and/or in the form of a suspension, solution, etc.

When the first compound and the second compound are combined in the form of a solution, the solution can be absorbed in porous carrier, such as porous matrix, porous solid particles, etc., the porous carrier containing the solution of first and second compounds can then be coated with a layer, such as an enterosoluble film layer, a gastric soluble layer, a controlled release layer, a layer for ensuring an extended release or a delayed release. The porous carrier containing the solution of first compound and second compound can be submitted to treatment, such as heat treatment for ensuring a deposit of first compound and/or second compound in the pores or on surface of the pores and/or for increasing the viscosity of the solution in the pores. The advantage of using such porous carrier containing the first and second compound in soluble form or in a substantially soluble form is that the release of first compound and second compound from the porous carrier occurs in the form of a solution, whereby facilitating the bioavailability of the active agent.

The porous carrier has for example a mean particle size of less than 5000 µm, such as less than 2000µm, advantageously less than 1000µm, for example a size comprised between 100µm and 800µm, such as an average particle size of 200µm, 300µm, 400µm, 500µm,600µm, 700µm and 750µm. The average pore diameter or average pore opening size is sufficient for enabling an easy passage of the solution into the pores, such diameter or size being for example lower than 20µm, such as lower than 10µm, advantageously lower than 2µm, preferably in average (average in number or average in volume) lower than 1µm, such as comprised between 5nanometer and 750nanometer, for example between 20nanometer and 600nanometer. The average size in volume can be estimated as being equal to (4 x total volume of the pores or porosity)/ (surface or specific surface). According to a specific embodiment, the average size in volume is determined by taking into account the pore volume formed by pore with a diameter or size greater than 2 nanometers, advantageously greater than 5 nanometers, and the specific surface or BET surface of the pores with a diameter or size greater than 2 nanometers, advantageously greater than 5 nanometers.

According to a still further possible embodiment, the combination further comprises at least one compound reacting in presence of water so as to prepare a substantially immediate solution of first compound and second compound. For example, the combination is an effervescent combination enabling the preparation of an aqueous solution containing the first and second compounds, in a very short time, substantially immediately, advantageously substantially without mechanical shaking.

The invention relates also to a pharmaceutical unit dose comprising at least a pharmaceutical combination containing at least:
- A first compound selected among the group consisting acetylsalicylic acid, salicylic acid, mixtures thereof, and
- A second compound selected from the group consisting of betaines, as defined previously, with the provision that said second compound is different from the first compound,

In which the combination is prepared from a mixture in which the first compound and the second compound are partly in a soluble form,

Whereby the pharmaceutical unit dose comprises less than 100 mg, advantageously less than 80 mg (such as less than 75 mg, preferably less than 50mg) of said first compound expressed as acetylsalicylic acid, and in which the amount of second compound is at least five times the amount, calculated as acetylsalicylic acid weight, of said first compound.

The combination of said unit dose is advantageously prepared from a mixture in which at least 50% by weight of the first compound and at least 50% of the second compound are in soluble form, preferably from a mixture in which at least 90% by weight of the first compound and at least 90% of the second compound are in soluble form, most preferably from a mixture in which the first compound and the second compound are substantially completely in soluble form.

The combination is for example in the form of dry particles, especially micro particles, prepared by drying a mixture in which the first compound and the second compound are partly in a soluble form.

A combination as an oral unit dose, wherein the first compound is in a form of a core in the form of dry particles, especially micro particles prepared by drying acetylsalicylic acid, salicylic acid, and mixtures thereof, and a second compound partly or completely in a soluble form selected from the group consisting of betaines, as defined previously said second compound in a amount at least five times by weight the amount of the first compound and partially or completely making a coating for the first compound.

The combination of the pharmaceutical unit dose of the invention can have one or more characteristics of the combination of the invention as disclosed here above.

The invention further relates to a kit for a daily administration, said kit comprising at least:
- An first oral formulation comprising at least a first compound selected among the group consisting acetylsalicylic acid, salicylic acid, and
- A second oral formulation comprising at least a second compound selected from the group consisting of betaines as defined before, with the provision that said second compound is different from the first compound
in which the first oral formulation comprises less than 100 mg of said first compound expressed as acetylsalicylic acid, and in which the amount of second compound in the second oral formulation is at least five times the amount, calculated as acetylsalicylic acid weight, of said first compound.

Advantageously, the first oral formulation comprises an amount of said first compound, calculated as acetylsalicylic acid, of less than 85 mg, advantageously of less than 75 mg, preferably of less than 60 mg. For example, the first oral formulation comprises an amount of acetylsalicylic acid corresponding to 5 to 80 mg, advantageously from 10 to 75 mg, preferably from 15 to 75 mg calculated as acetylsalicylic acid.

According to a specific embodiment, the second oral formulation comprises an amount of second compound corresponding to at least 10 times the amount, calculated as acetylsalicylic acid, of said first compound.

The kit is for example two distinct oral formulations to be administered successively immediately or to be administered at different moment of the day. For example, in the morning, the first oral formulation is administered, while at midday and/or in the afternoon and/or in the evening a second oral formulation is administered. The kit can thus comprise more than two oral formulations to be administered during a day. The first oral formulation can contain an amount of a second compound (different from the first compound), while the second oral formulation can contain an amount of a first compound (different from the second compound). According to a specific embodiment, the first oral formulation and the second oral formulation are substantially identical or similar. However, preferably the second oral formulation has a higher content of second compound and is poor in first compound. The kit can comprise more than 2 oral formulations, for example three, four, etc oral formulations for administering a one day dose. The oral formulations of a kit can be administered in different forms. For example a first oral administration to be taken as a dry formulation (tablet, pills, etc), while the second administration has to be taken as a syrup, solutions, etc.

Advantageously, the second oral formulation and/or third oral formulation comprise an amount of second compound corresponding to at least 20 times the amount, calculated as acetylsalicylic acid, of said first compound.

The first oral formulation comprises also an amount of a second compound selected from the group consisting of betaines as defined previously

with the provision that said second compound is different from the first compound.

For example, the first oral formulation is prepared at least from a mixture in which at least 50% by weight of the first compound and at least 50% of the second compound are in soluble form, advantageously at least from a mixture in which at least 90% by weight of the first compound and at least 90% of the second compound are in soluble form, preferably at least from a mixture in which the first compound and the second compound are substantially completely in soluble form.

Advantageously, the second oral formulation is at least a controlled release formulation for the second compound.

Preferably, the first oral formulation is at least an immediate release formulation for the first compound and possibly for an amount of the second compound.

According to a specific form of a kit of the invention requiring two oral administrations per day, the first administration is an oral administration of an oral formulation which is substantially an immediate release formulation for the first compound and for an amount of second compound different from the first compound (for example comprised between 0.5 and 10 times the amount of first compound, advantageously comprised between 1 and 5 times the amount of the first amount) and which is advantageously a controlled release for an amount of the second compound (for example release controlled for more than 8 hours, such as release controlled for 10 hours, 12 hours or even more), while the second oral formulation (for example to be taken 12 hours after the administration of the first oral formulation) is at least a controlled release formulation for the second compound (for example release controlled for more than 8 hours, such as release controlled for 12 hours, 24 hours or even more), said second oral formulation being preferably substantially free of first compound.

The use of
- A first compound selected among the group consisting of acetylsalicylic acid, salicylic acid, mixtures thereof, and
- A second compound selected from the group consisting of betaines as defined previously with the provision that said second compound is different from the first compound ,
for the preparation of a pharmaceutical combination for treating or preventing blood flow disturbances, and/or
for the preparation of a pharmaceutical combination for treating or preventing inflammation, and/or
for the preparation of a pharmaceutical combination for treating or preventing cancer, and/or
for the preparation of a pharmaceutical combination for treating or preventing diabetes and/or
for the preparation of a pharmaceutical combination for treating or preventing vascular diseases and/or
for the preparation of a pharmaceutical combination for treating or preventing at least one trouble related to aging and/or

The invention relates also to a pharmaceutical kit comprising at least one pharmaceutical combination of the invention or one pharmaceutical unit dose according to the invention , and a second pharmaceutical unit dose containing as active agent at least a compound selected from the group consisting of betaines as defined previously, with the provision that said second unit dose is free of compound selected among the group consisting acetylsalicylic acid, salicylic acid.

The invention further relates to a treatment of a patient in need for treating or for preventing thrombosis troubles for a patient, by administering to said patient a pharmaceutical combination according to the invention or a pharmaceutical unit dose according the invention, in which advantageously before and/or during and/or after said administration, a therapeutic effective amount of glycine betaine is administered to said patient for preventing or reducing the haemorrhagic side effect.

Acetylsalicylic acid, salicylic acid, and Betaines are administered orally, by tablets, capsules, syrup, etc. Administered doses can vary from 0.001g to 1 g per kg live body, for example from 0.005 g to 0,5 g, in particular from 0.01 g to 0,3 g per kg life body.

Examples of administration forms are: tablets, capsules, releasing forms, sublingual administration form, powder (for example for buccal inhalation), syrup, solution (nebulization, for example for buccal inhalation). As preferred administration form, entero soluble oral dosage form, such as gastro

insoluble tablets or capsules, etc. provided with an entero soluble coating or matrix or system.

The pharmaceutical combination can be in the form of a kit, so as to prepare the combination before administration or during the administration.

It is an object of the present invention to provide an oral controlled release of a betaine (as defined previously) preparation suitable for at least 5 minutes, such as for at least 10 minutes, ...twelve-hourly (e.g. up to twenty-four hourly or even more, such as for a week, two weeks, one month, three months) in combination with acetylsalicylic acid, salicylic acid, for the treatment of a mammal.

In accordance with the present invention, a pharmaceutical composition is provided which includes a betaine and aspirin, which provides for maximum patient benefits including maximum reduced risk of a myocardial infarction with minimal physical and chemical incompatibility (including minimal betaine/aspirin interaction), and reduced side effects normally associated with use of aspirin.

In addition, in accordance with the present invention, a treatment is provided for preventing or inhibiting or treating atherosclerosis, and/or reducing risk of or treating a cardiovascular event or disease including coronary artery disease and cerebrovascular disease, wherein a pharmaceutical composition containing a combination of a betaine and aspirin in a single dosage form, in a manner so as to minimize interaction of the betaine and aspirin, is administered to a patient in need of treatment.

Preferred pharmaceutical compositions of the present invention may take the form of several different embodiments. Thus, in one embodiment of the present invention, a pharmaceutical composition is provided wherein the betaine and aspirin are formulated together in a single tablet. The tablet of the invention is preferably in the form of a bilayered tablet which includes a first layer and a second layer. Aspirin, in the form of granules of preselected size will be present in the first layer together with optional excipients as described hereinafter, while the betaine will be present in the second layer which optionally may include one or more buffering agents (as necessary to prevent undesirable betaine/aspirin interaction) and optionally one or more excipients as described hereinafter. The betaine will be present in the second layer optionally in a slow and/or controlled release form.

In addition, the bilayered tablet of the invention may include an outer protective coating or finishing layer as described hereinafter.

In addition, the bilayered tablet of the invention may include further another therapeutically active agent.

In addition, the bilayered tablet of the invention may include further another therapeutically active agent as listed in the application PCT/BE 02/ 00013 of the one of the applicants.

Another embodiment of the present invention comprises a cored tablet which includes a core and a buffering layer or outer coat which can be compressed onto the core as a dry coat. The core will preferably include compressed aspirin granules while the buffering layer or outer coat will include a betaine together with one or more buffering agents and optional excipients.

Provision of aspirin in the core and betaine in the buffering layer will effectively reduce the aspirin dose needed, reduce its side effects and also minimize drug incompatibilities while providing maximum efficacy.

The so-described cored tablet may also optionally include an outer protective coating or finishing layer as described hereinafter.

In addition, in accordance with the present invention, a pharmaceutical composition is provided which is in the form of a tablet or capsule which includes a mixture of aspirin granules having an enteric coating and particles or granules of a betaine. Such a combination will provide maximum efficacy while minimizing side effects resulting from prolonged aspirin therapy.

In yet another embodiment of the pharmaceutical composition of the present invention, enteric coated aspirin granules as described above may be further coated with a protective coating or finishing layer. The double coated particles of aspirin can be mixed with Betaines as defined previously powders or granules, and the mixture can be encapsulated or tableted as described herein. This combination will protect the integrity of the enteric coat and minimize the side effects normally resulting from prolonged aspirin therapy. The aspirin and the betaine granules do not need to be mixed together; these can even be encapsulated separately into the same capsule shells in two shots.

Another embodiment of the pharmaceutical composition of the invention includes granules of enteric coated aspirin and enteric coated betaine, in the same dosage form such as compressed tablets or capsules.

The tablets containing the enteric coated granules of aspirin and betaine may also include an outer protective coating or finishing layer.

In a further embodiment of the pharmaceutical composition of the invention, where aspirin side effects are not an issue, for example, where low dose aspirin is present (80 mg or less), the composition of the invention may comprise a mixture of aspirin granules and betaine (including enteric coated particles of betaine or particles of betaine, containing an outer protective coating or finishing layer); the above mixture may take the form of compressed tablets or capsules (where the mixture can be encapsulated separately in two shots in the same capsule shells).

The pharmaceutical composition of the invention in the form of a tablet or capsule will include aspirin in amounts of less than 100mg.

The aspirin for use in forming the pharmaceutical composition of the invention will preferably be in the form of granules having an average particle size within the range from about 10 µm to about 2 mm, more preferably from about 0.25 mm to 1.0 mm.

The pharmaceutical composition of the invention will contain a Betaine as defined previously in an amount as normally employed for such betaine as exemplified in the patents thus, depending upon the particular betaine, it may be employed in amounts within the range from about 0.1 mg to 20000 mg per day in single or divided doses, and preferably from about 0.2 to about 10000 mg per day. Most preferably for betaine, a daily dosage in single or divided doses of 100 to 5000 mg, such as 300mg, 500mg, 750mg, 1000mg, 1500mg, 2000mg, 3000mg may be employed.

In forming the pharmaceutical composition of the invention in the form of a bilayered tablet, the first layer containing aspirin will also preferably include bulking agents such as lactose, microcrystalline cellulose, wood cellulose, corn starch, modified corn starch, calcium phosphate, sugar, dextrose, mannitol or sorbitol. The bulking agent will be present in an amount from about 1 to about 90%, preferably from about 5 to about 85% by weight of the first layer containing aspirin.

The first layer may also include a tabletting lubricant, such as zinc stearate, magnesium stearate, calcium stearate, talc, carnauba wax, stearic acid, palmitic acid or hydrogenated vegetable oils and fats, in an amount within the range from about 0.01 to about 4%, and preferably 0.02 to about 2% by weight of the first layer.

The second layer of the bilayered tablet containing betaine will usually include a bulking agent such as lactose, microcrystalline cellulose, modified corn starch, calcium phosphate or other bulking agent as set out above for the first layer, in an amount within the range from about 1 to about 90%, preferably from about 5 to about 85% by weight of the second layer. In addition, the second layer may include a binder such as corn starch, pregelatinized starch, polyvinyl pyrrolidone (PVP), hydroxypropylmethyl cellulose (HPMC), ethyl cellulose, cellulose acetate and the like, in an amount within the range from about 0.5 to about 20%, preferably from about 1 to about 10% by weight of the second layer, and a tabletting lubricant such as magnesium stearate, zinc stearate, or other lubricant as set out above with respect to the first layer in an amount from about 0.01 to about 4%, preferably from about 0.02 to about 2% by weight of the second layer.

The buffering agents present in the second layer may include conventional acid buffers such as calcium carbonate, magnesium oxide, magnesium carbonate, magnesium hydroxide, aluminum hydroxide, dihydroxyaluminum sodium carbonate, aluminum magnesium hydroxide sulfate or aluminum hydroxide magnesium carbonate co-dried gel, or mixtures of one or more thereof, in amounts as needed to insure that the aspirin will be sufficiently buffered to inhibit GI side effects. Thus, amounts of buffering agent within the range from about 10 to about 1000 mg, preferably from about 50 to about 500 mg will be employed depending upon the amount of aspirin present in the first layer.

In forming the bilayered tablet of the invention, the first layer containing aspirin may be prepared by conventional wet granulation or dry granulation (compaction) techniques.

The second layer containing betaine and buffers may be prepared by conventional wet granulation or dry granulation (compaction) techniques.

The first and second layers may then be compressed and combined to form a bilayered tablet employing conventional bilayer tabletting equipment.

Other conventional ingredients which may optionally be present in either of the two layers include preservatives, stabilizers, anti-adherents or silica flow conditioners or glidants, such as Syloid brand silicon dioxide as well as antioxidants such as Vitamin E, Vitamin C, and folic acid, Vitamin B.sub.6 and Vitamin B.sub.12.

The bilayer tablet of the invention may also include an outer protective coating layer which may comprise from 0 to about 15% by weight of the bilayer tablet. The outer protective coating layer which is applied over the bilayered tablet may comprise any conventional coating formulations and will include one or more film-formers or binders, such as a hydrophilic polymer like hydroxy-propylmethyl cellulose (HPMC) and a hydrophobic polymer like ethyl cellulose, cellulose acetate, polyvinyl alcohol-maleic anhydride copolymers, acrylic copolymers, beta.-pinene polymers, glyceryl esters of wood resins and the like, and one or more plasticizers, such as polyethylene glycol, triethyl citrate, diethyl phthalate, propylene glycol, glycerin, butyl phthalate, castor oil and the like.

The film formers are applied from a solvent system containing one or more solvents including water, alcohols like methyl alcohol, ethyl alcohol or isopropyl alcohol, ketones like acetone, or ethylmethyl ketone, chlorinated hydrocarbons like methylene chloride, dichloroethane, and 1,1,1-trichloroethane.

The pharmaceutical composition of the invention in the form of a cored tablet wherein the aspirin forms the core, and betaine plus buffering agent are present in a surrounding coat layer, may be prepared employing conventional cored tablet technology. Thus, the aspirin containing core (including excipients and other ingredients as described for the first layer in the bilayered tablet of the invention) may be formed in a manner similar to the first layer of the bilayered tablet as described hereinbefore. The buffering layer containing betaine as well as excipients and other ingredients (as described hereinbefore for the second layer of the bilayered tablet of the invention) may be compressed onto the core as a dry coat.

The so-formed cored tablet may be coated with an outer protective coating layer as described above for the bilayered tablet.

Another embodiment of the pharmaceutical composition of the invention is formed of tablets or capsules containing a mixture of enteric coated aspirin granules, and a betaine may be in the form of a tablet or capsule.

The aspirin granules can be coated with conventional enteric polymers coatings in aqueous or non-aqueous systems. For example, Eudragit L-30D-55 (acrylic acid copolymers-Rohm Pharma) (5 to 25% solids) containing 10 to 15% of diethylphthlate (w/w) as plasticizer can be used in an aqueous system.

Other conventional enteric polymer coating systems may be employed such as Eudragit R and S series resins, (acrylic acid copolymers-Rohm Pharma), cellulose acetate phthalate, hydroxypropylmethyl cellulose phthalate, hydroxypropylmethylcellulose acetate succinate, cellulose acetate maleate, cellulose acetate succinate and the like, and a suitable plasticizer such as triethyl citrate, diethyl phthalate, tributyl citrate, triacetin, dibutyl phthalate, dibutyl sebicate, Myvacet 940, and other commonly used plasticizers as may be suitable for particular enteric polymers can be used. It will be appreciated that any polymer with suitable plasticizer can be used in aqueous or non-aqueous system to form an enteric coating on the aspirin granule or particle.

In another embodiment of the pharmaceutical composition of the invention, the enteric coated aspirin granules described above may be further coated with an outer protective finishing coat or layer as described hereinbefore.

The double coated aspirin granules can be mixed with a betaine such as Betaines powders or granules and the mixture can be encapsulated or tableted as described above.

In yet another embodiment of the pharmaceutical composition of the invention, aspirin is enteric coated as described above and the betaine can optionally be enteric coated. The Betaines can be coated in the form of pure drugs or after spheronization or agglomeration. The particles for coating do not need to be perfectly spherical. These could be rods or irregular particles. The enteric coated particles of the two drugs (aspirin and betaine) can be tableted or encapsulated together. As described above, appropriate excipients (fillers, binders, disintegrants, and lubricant, etc.) can be used to facilitate tabletting. This betaine/aspirin combination will minimize side effects of aspirin, and eliminate chemical incompatibility.

If, aspirin side effects are not an issue, especially at lower (e.g., 80 mg) aspirin dosages, then aspirin granules (including uncoated aspirin) can be mixed with betaine powder or granules for tabletting or for encapsulating.

In yet another embodiment, aspirin granules can be mixed with enteric coated particles of betaine and the mixture can be tableted or encapsulated or the two granules can be encapsulated in two shots in the same capsule shells.

In carrying out the method of the present invention, the pharmaceutical composition of the invention containing the combination of the betaine and aspirin may be administered to mammalian species, such as monkeys, dogs, cattle, livestock, cats, rats, humans, etc., and, as described hereinbefore, may be incorporated in a tablet or capsule. The above dosage forms will also include the necessary carrier material, excipient, lubricant, buffer, antibacterial, bulking agent (such as mannitol), anti-oxidants such as Vitamin C and Vitamin E, as well as Vitamin B.sub.6, Vitamin B.sub.12, folic acid, sodium bisulfite, and the like.

The dose administered must be adjusted according to age, weight and condition of the patient, as well as the route of administration, dosage form and regimen and the desired result.

The compositions described above may be administered in the dosage forms as described above in single or divided doses of one to four times daily. It may be advisable to start a patient on a low dose combination and work up gradually to a high dose combination.

Tablets of various sizes can be prepared, e.g., of about 2 to 2000 mg in total weight, containing the active substances in the ranges described above, with the remainder being a physiologically acceptable carrier of other materials according to accepted pharmaceutical practice. These tablets can, of course, be scored to provide for fractional doses in some cases. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

In general, formulating the compositions, as described herein, the active substances, in the amounts described above, are compounded as described herein (according to accepted pharmaceutical practice) with a physiologically acceptable vehicle, carrier, excipient, binder, preservative, stabilizer, flavor, etc., in the particular type of unit dosage form.

Illustrative of the excipients which may be incorporated in tablets are the following: a binder such as gum tragacanth, acacia, corn starch or gelatin; an excipient such as dicalcium phosphate or cellulose; a disintegrating agent such as corn starch, potato starch, alginic acid, sodium starch glycolate or the like; a lubricant such as stearic acid, zinc stearate or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. As indicated, various other materials may be present as coatings or to otherwise modify the physical form of the dosage unit. For instance, tablets or capsules may be coated with shellac, sugar or both. A syrup of elixir may contain the active compounds, water, alcohol or the like as the carrier, glycerol as solubilizer, sucrose as sweetening agent, methyl and propyl parabens as preservatives, a dye and a flavoring such as cherry or orange.

Some of the active substances described above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for cardiovascular events and disease including coronary artery disease and/or cerebrovascular disease remains or the symptoms continue. Sustained release forms of such formulations which may provide such amounts daily, biweekly, weekly, monthly and the like may also be employed. A dosing period of at least 10 days are required to achieve minimal benefit.

The following examples illustrate various aspects of the present invention. They are not to be construed to limit the claims in any manner whatsoever.

In the following examples, embodiments having an amount of second compound of less than 5 times the amount of first compound of the combinations as claimed do not form part of the claimed invention and are included for illustrative purposes only. The same applies to embodiments or examples featuring betaine instead of glycine betaine anhydrous or -monohydrate.

### EXAMPLES

### Example 1

A bilayered tablet containing aspirin in a first layer and betaine in a second layer as described below may be prepared as follows.

| General Formula: | |
|---|---|
| | Amount or % |
| First Layer: | in First Layer |
| Aspirin granulation | 5 mg-300 mg |
| Lactose/microcrystalline | qs |
| Cellulose granulation* | |
| Zinc Stearate | 0.1 %-0.5 |

| | Amount in Second |
|---|---|
| Second Layer: | Layer |
| Calcium Carbonate | 5 mg-250 mg |
| Magnesium Oxide | 5mg-100 mg |
| Magnesium Carbonate | 2 mg-50 mg |
| Corn Starch | 5 mg-50 mg |
| Betaine | 100 mg-800 mg |
| Magnesium stearate | 0.2%-0.5% |

| | |
|---|---|
| *This is an inert granulation just for the purpose of bulking, if necessary. This will contain 50%-90% lactose anhydrous, 10%-50% microcrystalline cellulose, and 0.1%-0.5% zinc stearate. These ingredients are blended, and appropriate size granules are prepared by conventional dry granulation process. (This being just an inert granulation, any other excipient can be used to prepare granules for bulking by dry or wet granulation processes, # so that the granules do not have alkalizing agent and also do not contain excessive moisture and are compatible with aspirin granules. These bulking granules must have enough compatibility to allow compression of two layer tablets). | |

### Procedure

The aspirin granulation in the first layer is blended with sufficient quantity of the lactose/microcrystalline cellulose granulation as necessary to bulk up in order to have sufficient granulation to compress a satisfactory layer. The aspirin granules along with the bulking granules are blended with zinc stearate as a lubricant. Zinc stearate can be replaced with other non-alkaline lubricants, i.e., Lubritab.®. or other high melting point hydrogenated powdered waxes

Ingredients in the second layer are wet granulated using starch paste or other wet granulating materials, for example, PVP or HPMC, or can be dry granulated by compaction. The granules can be sized and lubricated. The two tablet layers are compressed using appropriate conventional tools and a suitable bilayer tabletting press, to form the bilayered tablet of the invention.

The quantity of the buffering agents used in the second layer can be adjusted as necessary to minimize gastrointestinal side effects. It should be understood that these buffering agents can be replaced with other suitable buffering agents, if desired.

The so-formed bilayered tablets may be coated with HPMC (hydroxypropylmethylcellulose) or commercially available Opadry.®. clear or Dri Klear.®. (HPMC) or any of these with any desired color. This coat is not limited to HPMC based coats only. Polymers, i.e., Eudragit E30D (acrylic acid copolymer) and others can also be used to give the tablets a finishing coat.

| Coating Formula (example): |
|---|
| Opadry .®. clear 10%-30% |
| Purified water qs |

### Procedure

Opadry.®. is dispersed in water to prepare a dispersion of 10%-30% solids*. This dispersion is used for coating the above tablets using conventional coating equipment. The coating of 0.2%-2% or any desired level (based on the weight of the finished coated bilayered tablet) can be applied to the bilayered tablet employing conventional techniques.
*Antifoam emulsion at a level of 0.1 to 2% of solids, can also be included in the formulation.

The so-formed tablets provide maximum benefits while minimizing drug interaction and other undesirable side effects.

It will be understood that betaine contained in the buffered layer of the bilayered tablet of the invention may be replaced with equivalent amounts of lipidic betaines and/or betaine lipids.

### Example 2

Tablets or capsules containing enteric coated aspirin and a betaine, which preferably is anhydrous betaine, monohydrate betaine, having the following composition are prepared as described below.

| | |
|---|---|
| General Formula: | |
| Aspirin particles | 5 mg-325 mg |
| Eudragit L-30D-55 | qs |
| Diethyl Phthalate | qs |
| Betaines, Desired Dose | |

### Procedure

Aspirin particles are coated with enteric polymers in aqueous or non-aqueous systems. Eudragit L-30D-55 containing 10%-15% of diethyl phthalate (w/w) is used in an aqueous system. The coating suspension is prepared having solid contents of 10%-30%.

To prepare the coating suspension, diethyl phthalate is added to the Eudragit L-30D-55 and the contents stirred till diethyl phthalate is completely dissolved. This is diluted with water to obtain the suspension with desired solid contents. Using this enteric coating suspension, the aspirin particles are coated in a fluid bed coating system using a Wurster insert or with top spray coating, so that aspirin particles of enteric quality can be produced. The enteric coated particles are mixed with betaine powders or granules and the mixtures are encapsulated or tableted using appropriate excipients (fillers, binder, disintegrants, and lubricants). Any of the listed betaine can be selected at its desired dose level along with the desired aspirin dose.

The Betaines can also be granulated, and the betaine granules and the enteric coated aspirin granules can be filled separately into the same capsule shell. Betaine granules can be prepared by dry or wet granulation processes, using suitable conventional excipients as is well known in the pharmaceutical field.

The above formulations provide maximum benefit while minimizing undesirable side effects and incompatibilities.

### Example 3

A cored tablet containing an aspirin core and a buffered coating thereon containing a betaine having the following composition is prepared as described below.

| General Formula: | |
|---|---|
| | Amount or % |
| Core Layer: | in Core Layer |
| Aspirin granulation | 10 mg-325 mg |
| Lactose/microcrystalline qs | |
| Cellulose granulation* | |
| Zinc Stearate | 0.1 %-0.5 |

| | Amount in Second |
|---|---|
| Outer Layer: | Layer |
| Calcium Carbonate | 5 mg-250 mg |
| Magnesium Oxide | 5 mg-100 mg |
| Magnesium Carbonate | 5 mg-50 mg |
| Corn Starch | 5 mg-50 mg |
| Betaine | 100 mg-1000 mg |
| Magnesium stearate | 0.2%-0.5% |
| Filler/Binder** | qs |

| | |
|---|---|
| *This is an inert granulation just for the purpose of bulking, if necessary. This will contain 50%-90% lactose anhydrous, 10%-50% microcrystalline cellulose, and 0.1%-0.5% zinc stearate. These ingredients are blended, and appropriate size granules are prepared by conventional dry granulation process. (This being just an inert granulation, any other excipient can be used to prepare granules for bulking by dry or wet granulation processes, # so that the granules do not have alkalizing agent and also do not contain excessive moisture and are compatible with aspirin granules. These bulking granules must have enough compatibility to allow compression of two layer tablets). **The Filler/Binder may be any known fillers or tablet binders, such as lactose, microcrystalline cellulose, modified starch, calcium phosphate and the like. | |

### Procedure

The aspirin granulation for the core is blended with sufficient quantity of the lactose/microcrystalline cellulose granulation as necessary to bulk up in order to have sufficient granulation to compress a satisfactory core. The aspirin granules along with the bulking granules are blended with zinc stearate as a lubricant. Zinc stearate can be replaced with other non-alkaline lubricants, i.e., Lubritab.®. or other high melting point hydrogenated powdered waxes.

Ingredients for the outer layer are wet granulated using starch paste or other wet granulating materials, for example, PVP or HPMC, or can be dry granulated by compaction. The granules can be sized and lubricated. The dry coated tablets can be compressed using appropriate tools and a suitable dry coating tabletting press.

The quantity of the buffering agents used in the outer layer can be adjusted as in Example 1. Other known buffering agents may be used as well.

The modes, the methods, the uses and the formulations as described in the application PCT/BE 02/ 00013 of one of the applicants, are also suitable for the Betaines / aspirin combinations in the scope of the present invention.

### Example 4

### Pharmacological activity in animal studies

### Appratus and methods

### Material

ASPEGIC® injectable aspirin Synthelabo France

Betaine monohydrate, BETAFIN ® ( Finnsugar Bioproducts, CULTOR, Helinski )
Rats Wistar, males, weight between 250 and 300 grams
Sodium Thiopental
Aggregometer CHRONOLOG COULTRONIC S.A. France
ADP Laboratoires Stago France

### Methods

### Aspirin dosage

The doses of 100 mg /kg and 50 mg /kg of aspirin used in these experiments are the doses known to be antithrombotic in rat in this model. Due to rat known resistance to aspirin these high doses are necessary and represent at least 10 to 25 fold the antithrombotic doses needed in other species in experimental thrombosis. Due to this specificity of rat the ratios betaine/aspirin used in these experiments don't reflect the ratio to be used in other species. In human clinical practice for instance, an orally daily dose of 75 to 300 mg, i.e. 1 to 5 mg/ kg is known to be antithrombotic. At this human dose (1 to 5 mg/ kg), betaine at 3 to 15 mg/kg or more, will be in accordance with the invention.

### Aggregation tests

The aggregation is made in accordance to the methods Cardinal & Flower. Pharmacol. Method.1980 and to American Journal of Clinical Pathology, 1989; 92: 676-679. Sureney. JD. Whole Blood aggregometry.

After a keeping period of 8 days, the rats are subjected to a fasting for 12 hours. Betaine is subcutaneous injected one hour before blood sampling. The rats are then anaesthetised with sodium Thiopental administered at a dose of 200 mg/Kg and the blood samples are taken by intracardiac puncture on a trisodium citrate solution (1 volume of solution at 3, 8 % citrate for 9 volumes of blood).

### Induced haemorrahgic time IHT

( E. Dejana. Bleeding time in rats. Thrombosis. Rech. 1982 )

Blood samples are made before the test. The tail of anaesthetised rat, is dipped for 5 minutes in a water bath at 37°C so as to provoke a dilatation of the peripheral vessels which are removed and cut at the end, the chronometer being started. The IHT is defined as being the time period comprised between the cutting of end tail and the end of the haemorrhage or bleeding. The end of haemorrhage is defined as the time where the last drop of blood is removed from the tail and no other drop is seen during 180 seconds. The substances were subcutaneously administrated 60 minutes prior to the tail cut.

### Principle of laser-induced thrombosis

### (Seiffge D. et al., 1989; Weichter W. et al., 1983)

In this model, lesion of the vascular wall is induced by a laser beam. This beam causes a limited lesion of the vascular endothelium (only 1 to 2 cells are destroyed). This laying bare of the sub-endothelium, which is a thrombogenetic surface, results in the adherence of platelets via glycoproteins. This adherence of platelets is followed by their activation, they form pseudopods and secrete the content of their granules. This activation results in the appearance of glycoprotein binding sites which are necessary for the aggregation of the platelets between them and for platelet adhesion to the thrombogenic surfaces. This lesion is induced in the mesenteric microcirculation of the rat. It is immediately followed by the formation of a thrombus (in a few seconds). This thrombus, which rapidly enlarges under the influence of the blood flow, embolises before being formed again.

### A. Aspirin 100 mg/kg / betaine 5 mg /kg combination

The aim of the study was to investigate the effect of betaine on various parameters in combination with aspirin.

### Experimental protocol.

The substances were subcutaneously injected 1 hour before the tests.

### Laser experiment

### (Saline control = 2 to 3 laser shoots & 5 to 6 emboli )

| | **Number of laser shoots** | | **Number of emboli** | |
|---|---|---|---|---|
| | ASA 100 mg /kg | ASA 100 mg /kg + betaine 5 mg /kg | ASA 100 mg /kg | ASA 100 mg /kg + betaine 5 mg /kg |
| Rat 1 | 4 | 4 | 0 | 0 |
| Rat 2 | 3 | 4 | 1 | 0 |
| Rat 3 | 3 | 4 | 1 | 0 |
| Rat 4 | 3 | 4 | 2 | 0 |
| Rat 5 | 3 | 4 | 2 | 0 |
| Rat 6 | - | 4 | - | 1 |
| **Mean** | **3,2 ± 0,45** | **4 ± 0** | **1,2 ± 0,84** | **0,17 ± 0,41** |

### Aggregation tests ( ADP 5 µM in final concentration )

### (Saline control = amplitude ± 15 ohm & velocity ± 13 ohm /min)

| | **Amplitude ( ohm )** | | **Velocity ( ohm /min )** | |
|---|---|---|---|---|
| | ASA 100 mg /kg | ASA 100 mg /kg + betaine 5 mg /kg | ASA 100 mg / kg | ASA 100 mg /kg + betaine 5 mg /kg |
| Rat 1 | 2 | 2 | 4 | 2 |
| Rat 2 | 3 | 0 | 4 | 0 |
| Rat 3 | 2 | 2 | 3 | 3 |
| Rat 4 | 4 | 0 | 5 | 1 |
| Rat 5 | 2 | 2 | 3 | 3 |
| Rat 6 | - | 2 | - | 3 |
| **Mean** | **2,6 ± 0,89** | **1,33 ± 1,03** | **3,8 ± 0,84** | **2 ± 1,26** |

### Induced haemorrhage (tail cut, Dejana)

### (Saline control = ± 110 seconds)

| | **IHT ( seconds )** | |
|---|---|---|
| | ASA 100 mg /kg | ASA 100 mg /kg + betaine 5 mg /kg |
| Rat 1 | 340 | 340 |
| Rat 2 | 330 | 320 |
| Rat 3 | 340 | 370 |
| Rat 4 | 345 | 345 |
| Rat 5 | 420 | 360 |
| Rat 6 | - | 320 |
| **Mean** | **355 ± 36,74** | **342 ± 20,43** |

### Discussion.

Betaine in combination with ASA had a little effect on induced haemorrhage. Surprisingly the combination shows better antithrombotic (more laser shoots & fewer emboli) effect than ASA alone, this being confirmed in the aggregation test. Betaine is potentialising aspirin effect, suggesting its possible activity on a different mechanism than aspirin. In this model the combination betaine / ASA showed better results than dipyridamole / aspirin combination suggesting a future clinical development.

### B. Aspirin 50mg/kg / betaine 10 mg /kg combination

Experimental protocol.
Betaine monohydrate Finnsugar, ASA Synthelabo
The substances were subcutaneously injected 1 hour before the tests.

### Laser experiment

Saline control NaCl 0, 9% subcutaneously 1 hour before experiments, duration of embolisation is expressed in minutes

| | Number of laser shoots | Number of emboli | Duration of embolisation |
|---|---|---|---|
| Rat 1 | 2 | 6 | 3 |
| Rat 2 | 2 | 5 | 2 |
| Rat 3 | 1 | 6 | 3 |
| Rat 4 | 1 | 7 | 4 |
| Rat 5 | 2 | 5 | 2 |
| Rat 6 | 1 | 6 | 4 |
| Mean | 1,5 | 5,83 | 3 |

Treated groups, ASA 50 mg /kg (n = 6 rats) or ASA 50 mg /kg + Betaine 10 mg/kg (n = 6 rats) are subcutaneously administrated 1 hour before experiments

| | Number of laser shoots | | Number of emboli | | Duration | |
|---|---|---|---|---|---|---|
| | ASA 50 mg /kg | ASA 50 + Betaine 10 | ASA 50 | ASA 50 + Betaine 10 | ASA 50 | ASA 50 + Betaine 10 |
| Rat 1 | 2 | 3 | 4 | 1 | 3 | 0 |
| Rat 2 | 3 | 2 | 3 | 1 | 2 | 0 |
| Rat 3 | 2 | 4 | 3 | 0 | 3 | 0 |
| Rat 4 | 2 | 4 | 4 | 0 | 4 | 0 |
| Rat 5 | 3 | 3 | 3 | 1 | 3 | 0 |
| Rat 6 | 2 | 4 | 3 | 0 | 4 | 0 |
| Mean | 2,33 | 3,33 | 3,33 | 0,5 | 2,5 | 0 |

### Aggregation tests (ADP 5 µM in final concentration )

### Amplitudes are expressed in Ohm and Velocities in Ohm /min.

| | Saline | | ASA 50 | | ASA 50 +Betaine 10 | |
|---|---|---|---|---|---|---|
| | Amplitude | velocity | Amplitude | velocity | Amplitude | velocity |
| Rat 1 | 15 | 14 | 8 | 7 | 2 | 2 |
| Rat 2 | 16 | 14 | 9 | 8 | 0 | 0 |
| Rat 3 | 15 | 13 | 10 | 9 | 1 | 0 |
| Rat 4 | 17 | 13 | 7 | 6 | 0 | 0 |
| Rat 5 | 16 | 15 | 9 | 7 | 2 | 3 |
| Rat 6 | 15 | 12 | 8 | 7 | 2 | 1 |
| Mean | 15,67 | 13,5 | 8,5 | 7,33 | 1,17 | 1 |

### Tail cut induced haemorrhage (Dejana)

| | Saline | ASA 50 | ASA 50 + Betaine 10 |
|---|---|---|---|
| Rat 1 | 98 | 183 | 114 |
| Rat 2 | 123 | 192 | 120 |
| Rat 3 | 107 | 176 | 127 |
| Rat 4 | 102 | 163 | 174 |
| Rat 5 | 114 | 185 | 111 |
| Rat 6 | 107 | 180 | 119 |
| Mean | 108,5 | 179,83 | 127,5 |

### Discussion.

Betaine in combination with ASA had a significant effect on induced haemorrhage while preserving the antithrombotic effect. The combination reducing by 2 times the therapeutic dose in rats (50 mg/kg instead of 100 mg/kg) shows better antithrombotic (more laser shoots & fewer emboli) effect than ASA alone at 100 mg/ kg, this being confirmed in the aggregation test. The combination of the invention clearly allows to reduce the effective dose of aspirin while obtaining a significant improvement in the therapeutic effect. Betaine is potentialising aspirin effect, suggesting its possible activity on a different mechanism than aspirin. The two molecules act in a synergistic manner to prevent thrombosis in this model. The combination betaine/ ASA showed better results than dipyridamole/ aspirin combination suggesting future clinical development.

These results show that glycine betaine maintains the tail cut bleeding time within the values of the negative control. In addition to its anti-thrombotic activity, the combination does not result in any risk of haemorrhage compared with the positive controls.

Treatment with asa/betaine combination completely inhibits the thrombo-embolic complications which are initiated by laser firings. In fact, treatment with asa/betaine combination before laser firings decreases the vascular adherence of platelets and the aggregation thereof.

Treatment with asa/betaine combination completely inhibits thrombo-embolic complications. In fact, treatment with asa/betaine combination before the induction of thrombosis exhibited a high antithrombotic potential with regard to all the parameters involved in thrombus formation process.

According the results presented above, this drug also exhibits anticoagulant, anti-aggregant and fibrinolytic indications. The demonstrated innocuousness of this combination enables long-term treatments to be considered which do not necessitate biological monitoring.

Interest in the use of asa/betaine combination is based on the fact that it acts at several levels of haemostatis, i.e. it acts on platelet aggregation, coagulation and fibrinolysis. This activity is durable and prevents repeated administration, which constitutes a considerable improvement in relation to existing treatments. The administration of betaine/asa does not induce any haemorrahgic risk or other side effects (e.g. heparin-induced thrombopenia), which constitutes a major advance in antithrombotic therapy.

### C. Aspirin 100 mg/kg / betaine 10 mg /kg combination

### Experimental protocol.

Betaine monohydrate Finnsugar, ASA Sigma Aldrich The substances were subcutaneously injected 1 hour before the tests.

### Laser experiment

Saline control NaCl 0, 9% subcutaneously 1 hour before experiments, duration of embolisation is expressed in minutes

| | **Number of laser shoots** | **Number of emboli** | **Duration of embolisation** |
|---|---|---|---|
| Rat 1 | 2 | 6 | 3 |
| Rat 2 | 2 | 5 | 2 |
| Rat 3 | 1 | 6 | 3 |
| Rat 4 | 1 | 7 | 4 |
| Rat 5 | 2 | 5 | 2 |
| Rat 6 | 1 | 6 | 4 |
| **Mean** | **1,5** | **5,83** | **3** |

Treated groups, ASA100 mg /kg (n = 6 rats) or ASA100 mg /kg + Betaine 10 mg/kg (n = 6 rats) are subcutaneously administrated 1 hour before experiments

| | **Number laser shoots** | | **Number of emboli** | | **Duration** | |
|---|---|---|---|---|---|---|
| | ASA100 mg /kg | ASA100 + Betaine 10 | ASA100 | ASA100 + Betaine 10 | ASA100 | ASA100 + Betaine 10 |
| Rat 1 | 2 | 4 | 2 | 0 | 1 | 0 |
| Rat 2 | 3 | 4 | 2 | 0 | 1 | 0 |
| Rat 3 | 3 | 4 | 1 | 0 | 0 | 0 |
| Rat 4 | 3 | 4 | 1 | 0 | 0 | 0 |
| Rat 5 | 4 | 4 | 0 | 0 | 0 | 0 |
| Rat 6 | 3 | 4 | 2 | 0 | 1 | 0 |
| **Mean** | 3 | 4 | 1,33 | 0 | 0,5 | 0 |

### Aggregation tests (ADP 5 µM in final concentration )

### Amplitudes are expressed in Ohm and Velocities in Ohm /min.

| | **Saline** | | **ASA100** | | **ASA100 +Betaine 10** | |
|---|---|---|---|---|---|---|
| | Amplitude | velocity | Amplitude | velocity | Amplitude | velocity |
| Rat 1 | 15 | 14 | 3 | 4 | 0 | 0 |
| Rat 2 | 16 | 14 | 3 | 3 | 0 | 0 |
| Rat 3 | 15 | 13 | 2 | 4 | 1 | 2 |
| Rat 4 | 17 | 13 | 2 | 2 | 0 | 0 |
| Rat 5 | 16 | 15 | 1 | 0 | 0 | 0 |
| Rat 6 | 15 | 12 | 4 | 4 | 1 | 0 |
| **Mean** | 15,67 | 13,5 | 2,5 | 2,83 | 0,33 | 0,33 |

### Tail cut induced haemorrhage in seconds (Dejana)

| | **Saline** | **ASA100** | **ASA100 +Bet. 10** |
|---|---|---|---|
| Rat 1 | 98 | 367 | 270 |
| Rat 2 | 123 | 413 | 290 |
| Rat 3 | 107 | 388 | 285 |
| Rat 4 | 102 | 397 | 240 |
| Rat 5 | 114 | 392 | 265 |
| Rat 6 | 107 | 368 | 250 |
| **Mean** | 108,5 | 387,5 | 266,7 |

### Discussion.

Betaine in combination with ASA had a significant effect on induced haemorrhage. The combination shows better antithrombotic (more laser shoots & fewer emboli) effect than ASA alone, this being confirmed in the aggregation test. Betaine is potentialising aspirin effect, suggesting its possible activity on a different mechanism than aspirin. In this model the combination betaine / ASA showed better results than clopidogrel/ aspirin combination confirming that this new combination in that ratio may be useful for parenterally administration in future acute clinical situations (i.e. angioplasties, prosthesis, stents placement, hip surgery, prosthetic heart valves, arterial grafts and replacement surgery).

### Example 5 :

### Pharmacological activity in human ex vivo test

### Parallel plate chamber studies

The synergistic effect of asa/betaine on platelet adhesion is studied by perfusing blood on thrombogenic surface such as collagen under shear stress conditions.

### Experimental design

Effect of betaine, aspirin or betaine/aspirin combination on platelet adhesion and thrombus formation on collagen coated surface.

We studied the effect of asa/betaine on platelet adhesion and thrombus formation under laminar flow at high shear stress (60 dynes /cm²) on a collagen coated surface. This high shear stress mimics stenosed arteries.
The blood of four fastened healthy subjects was sampled on day 1 as to determine basal values and betaine effect when added in vitro (table 1), then the subjects were orally administrated a daily oral bolus dose of 350 mg of ASA during 3 days and their blood sampled on day 3 as to determine ASA effect and ASA + betaine effect when betaine is added in vitro to aspirinized blood at different concentrations (table2).

Citrated human aspirinized blood was pre-labelled with mepacrine and exposed to vehicle alone or betaine (20, 40, and 80 µg/ml) for 20 minutes before perfusion.

### Effect of asa/betaine on platelet adhesion and thrombus formation on collagen coated surface.

The effect of asa/betaine has been evaluated by performing experiments 60 dynes/cm² witch mimics arterial thrombosis. Before perfusion citrated whole blood was incubated with saline alone or betaine at concentration of 20, 40, and 80 µg /ml for 20 minutes and then perfused on collagen at 60 dynes /cm². At the end of 2 minutes of perfusion, cells were fixed and the area covered by thrombi was calculated by analysis of fluorescent thrombus images acquired by confocal microscopy.

### Experimental Methods

Collagen coated slides. Glass slides were with collagen (200µg/ml; equine collagen), placed for 1 hour in a water bath at 37°C, and the collagen gel allowed to evaporate for 30 minutes under a ventilated hood. Perfusion with citrated whole blood was started and continued for 2 minutes, then blood was withdrawn from the circuit, and platelet thrombi adherent to the collagen coated surface were fixed and stained with May Grunwald-Giemsa.
Images of platelet thrombi were digitised using a light microscope connected to a computer-based image analysis system. The surface occupied by thrombi and mean thrombus area were evaluated by automatic edge detection using built-in specific functions of a software image.
Platelet adhesion and thrombus formation. Platelet adhesion assay was performed according to the described previously (Alevriadou et al.,1993; Boccardo et al.,1997) with minor modifications. Blood collected on citrate (3,8 %) was perfused through a flow chamber using a syringe pump. A flow chamber thermostatized to 37°C was used in which one surface of the perfusion channel is a glass slide coated with equine collagen.

Flow chamber. The chamber dimensions (30 mm long, 1mm large, and 150 µm in the thickness) allow to obtain a wide range of shear rate low flow rates of blood.

### Adhesion assay collagen.

Citrated whole blood was incubated with the fluorescent dye mepacrine 10 µM (quinacrine dihydrochloride BP; Sigma Chemical, St Louis. MO). Mepacrine concentrated in the dense granules of platelets and in the granules of leukocytes and, at this concentration has no effect on normal platelet function (3). Any fluorescence within the erythrocytes is quenched by haemoglobin. Blood was pre-incubated 5 minutes at 37°C before perfusion. The system was filled with PBS pH 7.3, then perfusion with blood was started and maintained at the constant flow rate of 1500 sec⁻¹ (corresponding to a shear stress of 60 dynes/cm²). After 2 min, perfusion was stopped and the slide with the collagen monolayer was dehydrated and fixed in acetone for 20 minutes.
Images of platelet thrombi on collagen surface were acquired by a confocal inverted laser microscope. The surface occupied by thrombi was evaluated by automatic edge detection using built-in specific functions of a software image.

### Results

### 1) Effect of betaine on platelet adhesion and thrombus formation on collagen.

Thrombus formation on collagen coated surface studied at the shear stress of 60 dynes /cm² in which Von Willebrand factor is involved. The effect of betaine added to non-stimulated blood perfused on collagen at high shear stress (60 dynes/cm²) shows a dose-dependent inhibitory activity of asa/betaine. As shown in Table 1, blood treated with betaine at the concentration of 80 µg/ml showed a significant reduction (- 49 %) in platelet deposition in respect to vehicle. Thrombus formation of blood incubated with asa/betaine at 20 and 40 µg /ml was inhibited by 15 and 41 % respectively

### 2) Effect of asa/betaine on thrombus formation on collagen.

Thrombus formation on collagen coated surface studied at the shear stress of 60 dynes /cm² in which Von Willebrand factor is involved. The effect of betaine and asa/betaine added to non-stimulated blood perfused on collagen at high shear stress (60 dynes/cm²) shows a dose-dependent inhibitory activity of asa/betaine. As shown in Table 2, aspirinized blood treated with betaine at the concentration of 80 µg/ml showed a significant reduction ( - 63 % ) in platelet deposition in respect to vehicle and a significant reduction in respect to aspirin alone (- 51 % ) and to betaine alone (- 27 % ).

**Table 1 :**

| Subject | saline | betaine | betaine | betaine |
|---|---|---|---|---|
| | | 20 µg/ml | 40 µg/ml | 80 µg/ml |
| 1 | 100586 | 88746 | 66852 | 57544 |
| 2 | 145921 | 135241 | 92433 | 81585 |
| 3 | 165239 | 122369 | 89628 | 76654 |
| 4 | 175692 | 154425 | 95645 | 82579 |
| **Mean** | **146859** | **125195** | **86139** | **74590** |

Data are expressed as area covered by thrombi (pixels/field)
Blood was incubated with saline or betaine for 20 minutes **Table 2:**

| Subject | saline | ASA + | ASA +betaine | ASA +betaine | ASA +betaine |
|---|---|---|---|---|---|
| | | saline | 20 µg/ml | 40 µg/ml | 80 µg/ml |
| 1 | 100586 | 72856 | 65439 | 59422 | 45253 |
| 2 | 145921 | 121321 | 121822 | 76754 | 55114 |
| 3 | 165239 | 132458 | 104583 | 69575 | 55752 |
| 4 | 175692 | 122652 | 106869 | 85856 | 62687 |
| **Mean** | **146859** | **112322** | **99678** | **72857** | **54701** |

Data are expressed as area covered by thrombi (pixels/field)
Aspirinized blood was incubated with saline or betaine for 20 minutes

### Comments

In this assay we analysed the potential antithrombotic effect of betaine and asa/betaine on thrombus formation under controlled flow conditions. We observed that under shear stress level high enough to mimic the one encountered in the microcirculation, betaine and asa/betaine significantly inhibit platelet deposition and consequent thrombus formation with respect to vehicle-treated blood. The combination asa/betaine was more effective than aspirin alone or betaine. These tests clearly show the synergistic and addictive effect of the 2 molecules, namely betaine and aspirin, when used in combination in an human ex vivo experimental thrombosis model. These results are predictive of clinical efficacy of the claimed combinations.

### Uses of the invention

In view of the above specifications, the invention relates thus also to:
- the use of a one "Betaines" mixtures thereof and acetylsalicylic acid, salicylic acid and/or mixtures thereof for the preparation of a pharmaceutical composition for the treatment or the prevention of troubles bound to one or more glycoproteins, especially to receptor of one or more glycoproteins, preferably to receptors of glycoproteins Ib and IIb IIIa.
- the use of Betaines mixtures thereof and acetylsalicylic acid, salicylic acid and/or mixtures thereof for the preparation of a pharmaceutical composition for the treatment or the prevention of troubles bound to one or more glycoproteins, especially to receptor of one or more glycoproteins, preferably to receptor of glycoprotein IIb IIIa for inhibiting the platelet aggregation.
- the use of Betaines mixtures thereof and acetylsalicylic acid, salicylic acid and/or mixtures thereof for the preparation of a pharmaceutical composition for the treatment or the prevention of troubles bound to one or more glycoproteins, especially to receptor of one or more glycoproteins, preferably to receptor of glycoprotein IIb IIIa for avoiding the adhesion of cells there between
- pharmaceutical composition comprising insulin and Betaines mixtures thereof and acetylsalicylic acid, salicylic acid and/or mixtures thereof
- pharmaceutical composition comprising an anti cancerous agent and at least Betaines, mixtures thereof and acetylsalicylic acid, salicylic acid and/or mixtures thereof
- pharmaceutical composition comprising an antibiotic and at least Betaines, mixtures thereof and acetylsalicylic acid, salicylic acid and/or mixtures thereof
- use of Betaines, mixtures thereof and acetylsalicylic acid, salicylic acid and/or mixtures thereof, as glycoproteic antagonist agent, in particular as antagonist of the glycoprotein Ib and/ or glycoprotein IIb IIIa, for the preparation of a pharmaceutical composition
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to cancer, in particular to the metastasis of cancerous cells
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to blood circulation, in particular to the blood microcirculation
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to chemotherapy
- use of Betaines, mixtures thereof and acetylsalicylic acid, salicylic acid and/or mixtures thereof, as therapeutic active agent for the preparation of a pharmaceutical composition for the treatment or the prevention or the stabilization of diabetic troubles
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to aging
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to oestrogen oral contraception
- use of Betaines, mixtures thereof and acetylsalicylic acid, salicylic acid and/or mixtures thereof, as therapeutically active agent for the preparation of a pharmaceutical composition for the treatment or the prevention or the stabilization of troubles bound to extracorporeal blood circulation, in particular to troubles bound to dialysis and to haemodialysis
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to inflammation, in particular internal inflammation troubles
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to bites, in particular to bites of venomous animals,
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to post traumatic shock or post surgical shock,
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to septic shocks,
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to embolism, in particular to cerebral embolism and/or pulmonary embolism
- use of Betaines, mixtures thereof and acetylsalicylic acid, salicylic acid and/or mixtures thereof, as therapeutic active agent

for the preparation of a pharmaceutical composition for the treatment or the prevention or the stabilization of troubles bound to an infract
- use of Betaines, mixtures thereof and acetylsalicylic acid, salicylic acid and/or mixtures thereof, as therapeutically active agent for the preparation of a pharmaceutical composition for the treatment or the prevention or the stabilization of troubles bound to aneurysm
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to phlebitis
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to angina pectoris
- use of Betaines, mixtures thereof and acetylsalicylic acid, salicylic acid and/or mixtures thereof, as therapeutic active agent for the preparation of a pharmaceutical composition for the treatment or the prevention or the stabilization of thromboses troubles, in particular troubles bound to reocclusion of the vascular system and/or to thrombolysis and/or to angioplasty
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to the use of hemoplastic or haemostatic glues, in particular fibrinogen glue, fibrin glue, collagen glue, thrombin glue
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to pregnancy
- use of Betaines, mixtures thereof and acetylsalicylic acid, salicylic acid and/or mixtures thereof, as therapeutic active agent for the preparation of a pharmaceutical composition for the treatment or the prevention or the stabilization of thromboses troubles, in particular coronary thrombosis and/or venous thrombosis
- use of Betaines, mixtures thereof and acetylsalicylic acid, salicylic acid and/or mixtures thereof, as therapeutic active agent for the preparation of a pharmaceutical composition for the treatment or the prevention or the stabilization of bacterial troubles and/or infectious troubles and/or troubles due to virus and/or troubles due to fungus
- use of Betaines, mixtures thereof and acetylsalicylic acid, salicylic acid and/or mixtures thereof, as therapeutic active agent for the preparation of a pharmaceutical composition for the treatment or the prevention or the stabilization of asthmatic troubles
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to osteoporosis
- use of Betaines, mixtures thereof and acetylsalicylic acid, salicylic acid and/or mixtures thereof, as therapeutic active agent for the preparation of a pharmaceutical composition for the treatment or the prevention or the stabilization of troubles bound to graft of skin and/or tissue and/or bone and/or cells
- use of Betaines, mixtures thereof and acetylsalicylic acid, salicylic acid and/or mixtures thereof, as antagonist agent for serotinin and/or arachidonic acid and/or epinephrine and/or adrenaline and/or thrombin and/or ristocetine for the preparation of a pharmaceutical composition
- uses as disclosed here before for the preparation of a pharmaceutical form, possibly as a kit, containing an active agent different from Betaines, mixtures thereof and acetylsalicylic acid, salicylic acid and/or mixtures thereof, for the administration (simultaneous or successive, with the same or different administration path) of said other therapeutic active agent and of Betaines, mixtures thereof and acetylsalicylic acid, salicylic acid and/or mixtures thereof
- Sweetening composition containing at least a sweetener and at least Betaines, mixtures thereof and acetylsalicylic acid, salicylic acid and/or mixtures thereof
- Sweetening composition containing at least Betaines, mixtures thereof and acetylsalicylic acid, salicylic acid and/or mixtures thereof
- use of Betaines, mixtures thereof and acetylsalicylic acid, salicylic acid and/or mixtures thereof, as agent for improving the sweetening property of a sweetener, in particular of a synthetic sweetener
- Preserving process for cells and/or platelets in a medium, in particular in a blood medium or a fraction thereof, in which said medium is added or mixed with Betaines, mixtures thereof and acetylsalicylic acid, salicylic acid and/or mixtures thereof
- Device with a surface in contact with fibrin and/or fibrinogen and/or collagen, said surface being made of and being treated with a composition containing at least Betaines, mixtures thereof and acetylsalicylic acid, salicylic acid and/or mixtures thereof
- Artificial device destined to be implanted to a living body with a surface in contact with blood said surface being made of and/or being treated with a composition containing at least Betaines, mixtures thereof and acetylsalicylic acid, salicylic acid and/or mixtures thereof
- Artificial device destined to be implanted to a living body with a surface in contact with living tissue said surface being made of and/or being treated with a composition containing at least Betaines, mixtures thereof and acetylsalicylic acid, salicylic acid and/or mixtures thereof
- Composition containing at least fibrinogen and at least Betaines, mixtures thereof and acetylsalicylic acid, salicylic acid and/or mixtures thereof
- Composition containing at least collagen and at least Betaines, mixtures thereof and acetylsalicylic acid, salicylic acid and/or mixtures thereof
- Process for the treatment of blood or a fraction thereof and acetylsalicylic acid, salicylic acid, salicylates and derivatives and/or mixtures thereof by osmosis and/or reverse osmosis, in which, before and/or during and/or after the osmosis or reverse osmosis, said blood is added or mixed with at least Betaines, mixtures thereof and acetylsalicylic acid, salicylic acid and/or mixtures thereof
- Process for the treatment of blood or a fraction thereof and acetylsalicylic acid, salicylic acid and/or mixtures thereof by centrifugation, in which, before and/or during and/or after the centrifugation, said blood is added or mixed with at least Betaines, mixtures thereof and acetylsalicylic acid, salicylic acid and/or mixtures thereof
- Biological material or synthetic material for implant purposes, especially for bone implant, said material being treated with Betaines, mixtures thereof and acetylsalicylic acid, salicylic acid and/or mixtures thereof, and/or a composition containing such a compound
- Process of treatment of a patient suffering of a trouble cited here above in this specification, in which an effective amount of Betaines, mixtures thereof and acetylsalicylic acid, salicylic acid and/or mixtures thereof is administered to said patient, so as to treat and/or stabilize said trouble
- Process for preventing a patient to suffer a trouble cited here above in this specification, in which an effective amount of Betaines, mixtures thereof and acetylsalicylic acid, salicylic acid and/or mixtures thereof is administered to said patient, so as to prevent said trouble,
- use of Betaines, mixtures thereof and acetylsalicylic acid, salicylic acid and/or mixtures thereof, as anti agglutinant agent, for the preparation of a pharmaceutical composition
- use of Betaines, mixtures thereof and acetylsalicylic acid, salicylic acid and/or mixtures thereof, as blood fluidifying agent, for the preparation of a pharmaceutical composition.

Aspirin will preferably be employed in the form of salicylic acid acetate also referred to as acetylsalicylic acid.

In one embodiment salicylic acid may be employed.

Salicylate salts may also be employed.

In one embodiment, the combinations of the invention will be useful for the treatment of pain, inflammation, and fever rheumatoid arthritis, osteoarthritis, juvenile and adult forms of arthritis, gout, dysmenorrhea, muscle pains, and dental pain.

In one embodiment, the modes, the methods, the combinations, the uses and the formulations as described in the application PCT/BE 02/00013 of one of the applicants, are also suitable for the Betaines/ aspirin combinations when formulated or combined with antithrombotic, anti inflammatory, anti cancerous and anti diabetic drugs.

In one embodiment, the modes, the methods, the combinations, the uses and the formulations as described in the application PCT/BE 02/00013 of one of the applicants are also suitable for the Betaines/ aspirin combinations when Betaines are formulated in a slow or controlled release forms.

The combinations of the invention may be useful for long term therapies as vascular occlusive diseases, inflammation, cancer and diabetes.

## Claims

1. Oral Pharmaceutical combination comprising at least:
- A first compound selected among the group consisting of acetylsalicylic acid, salicylic acid, and pharmaceutical salts thereof, and
- A second compound selected from the group consisting of glycine betaine anhydrous and glycine betaine monohydrate,
in which said combination comprises less than 100 mg of said first compound expressed as acetylsalicylic acid, and
in which the amount of second compound is at least 5 times the amount, calculated as acetylsalicylic acid weight, of said first compound.

2. The combination of claim 1, which comprises an amount of said first compound, calculated as acetylsalicylic acid, of less than 85 mg, advantageously of less than 75 mg, preferably of less than 60 mg.

3. The combination of claim 1, which comprises an amount of acetylsalicylic acid or pharmaceutical salt thereof corresponding to 3 to 80 mg, advantageously from 5 to 75 mg, preferably from 10 to 75 mg calculated as acetylsalicylic acid.

4. The combination of claim 1, in which the amount of second compound is comprised between 5 and 25 times the amount calculated as acetylsalicylic acid weight of said first compound.

5. The combination of claim 1, which is prepared at least from a mixture in which at least 50% by weight, preferably at least 90% by weight of the first compound and at least 50% by weight, preferably at least 90% by weight of the second compound are in soluble form.

6. The combination of claim 1, in which the second compound is at least in a controlled release form and/or in which the first compound is at least partly in an immediate release form.

7. The combination of claim 1, in which the first compound is at least partly in an immediate release form.

8. The combination of claim 1, which comprises dry particles, especially micro particles, prepared by drying a mixture in which the first compound and the second compound are partly in a soluble form.

9. The combination of claim 1, in which the first compound and the second compound are combined in the form selected from the group consisting of a matrix, a gel, an hydrogel, a wax and a porous carrier, a bilayered tablet and combination thereof.

10. Oral Pharmaceutical unit dosage form comprising at least a pharmaceutical combination of any one of the claims 1 to 9.

11. A kit for a daily administration, said kit comprising at least:
- An first oral formulation comprising a first compound selected among the group consisting of acetylsalicylic acid, salicylic acid, and pharmaceutical salts thereof, and
- A second oral formulation comprising a second compound selected from the group consisting of glycine betaine anhydrous and glycine betaine monohydrate, in which the first oral formulation comprises less than 100 mg of said first compound expressed as acetylsalicylic acid, and
in which the amount of second compound in the second oral formulation is at least five times the amount, calculated as acetylsalicylic acid, of said first compound.

12. The kit of claim 11, in which the first oral formulation comprises an amount of said first compound, calculated as acetylsalicylic acid, of less than 85 mg, advantageously of less than 75 mg, preferably of less than 60 mg.

13. The kit of claim 12, which is adapted for the preparation of a combination according to any one of the claims 2 to 9.

14. The use of
- a first compound selected among the group consisting of acetylsalicylic acid, salicylic acid, pharmaceutical salts thereof, and
- a second compound selected from the group consisting of glycine betaine anhydrous and glycine betaine monohydrate
for the preparation of an oral medicament according to anyone of the claims 1 to 9 or a pharmaceutical dosage form according to claim 10 or a kit according to any one of the claims 11 to 13, for treating or preventing blood flow disturbances or
treating or preventing cancer or
for treating or preventing diabetes, or
for treating or preventing gut troubles, or
for treating or preventing inflammation.

15. Oral pharmaceutical composition which is a pharmaceutical combination according to any one of the claims 1 to 9, said composition comprising a betaine and aspirin in a formulation wherein the betaine and aspirin are formulated together in a bilayered tablet, the aspirin being present in a first layer, and the betaine being present in a second layer in an amount at least five times the amount of aspirin.

16. The pharmaceutical composition as defined in claim 15, wherein the layer containing the betaine also includes one or more buffering agents.

17. The pharmaceutical composition as defined in claim 15, wherein the tablet includes a core and a coating layer surrounding said core and wherein one of the betaine and aspirin is present in the core and the other is present in a coating layer surrounding the core.

18. The pharmaceutical composition as defined in claim 15, wherein the tablet includes a core and a coating layer surrounding said core and wherein a mixture of the betaine and aspirin is present in the core and one of the betaine and aspirin is present in the coating layer surrounding the core.

19. The pharmaceutical composition as defined in claim 17, wherein the aspirin is present in the core and the betaine is present in the coating layer.

20. The pharmaceutical composition as defined in anyone of the claims 15 to 19, wherein the aspirin is present in the core and the betaine present in the coating layer is in a controlled release form.

21. The pharmaceutical composition as defined in anyone of the claims 15 to 20, wherein the betaine is present in the core in a controlled release form and the aspirin is present in the coating layer.

22. The pharmaceutical composition as defined in claim 21 wherein the coating layer also includes one or more buffering agents.

23. The pharmaceutical composition as defined in claim 22 wherein the coating layer also includes one or more buffering agents and one or more protecting films.

24. The pharmaceutical composition as defined in claim 23 wherein the betaine is selected from the group consisting of glycine betaine anhydrous and glycine betaine monohydrate.

25. The pharmaceutical composition as defined in claim 24 further including an outer protective coating or finishing layer surrounding said tablet.

26. The pharmaceutical composition as defined in claim 15 wherein the aspirin is in the form of enteric coated aspirin granules.

27. The pharmaceutical composition as defined in claim 1 in the form of a bilayered tablet which comprises a first layer comprising aspirin granules and one or more excipients, and a second layer comprising a betaine and one or more buffering compounds and one or more excipients.

28. The pharmaceutical composition as defined in claim 27, wherein the first layer comprises aspirin granules, one or more bulking agents and optionally a lubricant, and the second layer comprises a betaine, optionally a wet granulating agent, one or more buffering compounds selected from the group consisting of calcium carbonate, magnesium oxide, magnesium carbonate and mixtures thereof, and optionally magnesium stearate.

29. The pharmaceutical compositions as defined in anyone of the claims 15 to 28 further including an antithrombotic agent or an anti cancerous agent or an anti inflammatory agent or an antibiotic agent or an anti diabetic agent or an antioxidant agent.

30. The combination of claim 1, in which the amount of second compound is at least 20 times for instance, 30, 40, 50, 70, 85 or 100 times the amount calculated as acetylsalicylic acid weight of said first compound.

## Patentansprüche

1. Orale pharmazeutische Kombination umfassend mindestens
- eine erste Verbindung, die ausgewählt ist aus der Gruppe bestehend aus Acetylsalicylsäure, Salicylsäure und pharmazeutischen Salzen davon, und
- eine zweite Verbindung, die ausgewählt ist aus der Gruppe bestehend aus Glycin-wasserfreiem Betain und Glycin-Betainmonohydrat,
wobei die Kombination weniger als 100 mg der ersten Verbindung, ausgedrückt als Acetylsalicylsäure, umfasst, und
wobei die Menge der zweiten Verbindung mindestens die fünffache Menge, berechnet als Gewicht der Acetylsalicylsäure, der ersten Verbindung ist.

2. Kombination nach Anspruch 1, die eine Menge der ersten Verbindung, berechnet als Acetylsalicysäure, von weniger als 85 mg, vorteilhaft von weniger als 75 mg, vorzugsweise von weniger als 60 mg umfasst.

3. Kombination nach Anspruch 1, die eine Menge von Acetylsalicylsäure oder eines pharmazeutischen Salzes davon umfasst, die 3 bis 80 mg, vorteilhaft 5 bis 75 mg, vorzugsweise 10 bis 75 mg, berechnet als Acetylsalicylsäure, entspricht.

4. Kombination nach Anspruch 1, wobei die Menge der zweiten Verbindung zwischen dem 5- und 25-Fachen der Menge, berechnet als Gewicht der Acetylsalicylsäure, der ersten Verbindung liegt.

5. Kombination nach Anspruch 1, die aus mindestens einem Gemisch hergestellt ist, in dem mindestens 50 Gewichtsprozent, vorzugsweise mindestens 90 Gewichtsprozent der ersten Verbindung und mindestens 50 Gewichtsprozent, vorzugsweise mindestens 90 Gewichtsprozent der zweiten Verbindung in löslicher Form sind.

6. Kombination nach Anspruch 1, wobei die zweite Verbindung mindestens in einer Form mit kontrollierter Freisetzung ist und/oder wobei die erste Verbindung mindestens teilweise in einer Form mit unmittelbarer Freisetzung ist.

7. Kombination nach Anspruch 1, wobei die erste Verbindung mindestens teilweise in einer Form mit unmittelbarer Freisetzung ist.

8. Kombination nach Anspruch 1, die trockene Partikel, insbesondere Mikropartikel umfasst, die durch Trocknen eines Gemisches hergestellt werden, wobei die erste Verbindung und die zweite Verbindung teilweise in einer löslichen Form sind.

9. Kombination nach Anspruch 1, wobei die erste Verbindung und die zweite Verbindung in der Form kombiniert sind, die ausgewählt ist aus der Gruppe bestehend aus einer Matrix, einem Gel, einem Hydrogel, einem Wachs und einem porösen Träger, einer zweischichtigen Tablette und einer Kombination davon.

10. Orale pharmazeutische Einheitsdosierungsform, umfassend mindestens eine pharmazeutische Kombination nach einem der Ansprüche 1 bis 9.

11. Kit für eine tägliche Verabreichung, wobei der Kit mindestens Folgendes umfasst:
- eine erste orale Formulierung, umfassend eine erste Verbindung, die ausgewählt ist aus der Gruppe bestehend aus Acetylsalicylsäure, Salicylsäure und pharmazeutischen Salzen davon, und
- eine zweite orale Formulierung, umfassend eine zweite Verbindung, die ausgewählt ist aus der Gruppe bestehend aus Glycin-wasserfreiem Betain und Glycin-B etainmonohydrat,
wobei die erste orale Formulierung weniger als 100 mg der ersten Verbindung, ausgedrückt als Acetylsalicysäure, umfasst, und
wobei die Menge der zweiten Verbindung in der zweiten oralen Formulierung mindestens fünfmal die Menge, berechnet als Acetylsalicylsäure, der ersten Verbindung ist.

12. Kit nach Anspruch 11, wobei die erste orale Formulierung eine Menge der ersten Verbindung, berechnet als Acetylsalicylsäure, von weniger als 85 mg, vorteilhaft von weniger als 75 mg, vorzugsweise von weniger als 60 mg, umfasst.

13. Kit nach Anspruch 12, der für die Herstellung einer Kombination nach einem der Ansprüche 2 bis 9 ausgebildet ist.

14. Verwendung
- einer ersten Verbindung, die ausgewählt ist aus der Gruppe bestehend aus Acetylsalicylsäure, Salicylsäure, pharmazeutischen Salzen davon, und
- einer zweiten Verbindung, die ausgewählt ist aus der Gruppe bestehend aus Glycin-wasserfreiem Betain und Glycin-Betainmonohydrat, für die Herstellung eines oralen Medikaments nach einem der Ansprüche 1 bis 9 oder einer pharmazeutischen Dosierungsform nach Anspruch 10 oder eines Kits nach einem der Ansprüche 11 bis 13, zur Behandlung oder Prävention von Durchblutungsstörungen, oder
Behandlung oder Prävention von Krebs, oder
zur Behandlung oder Prävention von Diabetes, oder
zur Behandlung oder Prävention von Darmbeschwerden, oder
zur Behandlung oder Prävention einer Entzündung.

15. Orale pharmazeutische Zusammensetzung, die eine pharmazeutische Kombination nach einem der Ansprüche 1 bis 9 ist, wobei die Zusammensetzung ein Betain und Aspirin in einer Formulierung umfasst, wobei das Betain und Aspirin gemeinsam in einer zweischichtigen Tablette formuliert sind, wobei das Aspirin in einer ersten Schicht vorhanden ist und das Betain in einer zweiten Schicht in einer Menge vorhanden ist, die mindestens die fünffache Menge des Aspirins ist.

16. Pharmazeutische Zusammensetzung nach Anspruch 15, wobei die Schicht, die das Betain enthält, auch ein oder mehrere Puffermittel enthält.

17. Pharmazeutischen Zusammensetzung nach Anspruch 15, wobei die Tablette einen Kern und eine Überzugsschicht enthält, die den Kern umgibt, wobei eines von Betain und Aspirin in dem Kern und das andere in einer Überzugsschicht enthalten ist, die den Kern umgibt.

18. Pharmazeutische Zusammensetzung nach Anspruch 15, wobei die Tablette einen Kern und eine Überzugsschicht enthält, die den Kern umgibt, wobei ein Gemisch aus dem Betain und Aspirin in dem Kern vorhanden ist und eines von dem Betain und Aspirin in der Überzugsschicht enthalten ist, die den Kern umgibt.

19. Pharmazeutische Zusammensetzung nach Anspruch 17, wobei das Aspirin in dem Kern vorhanden ist und das Betain in der Überzugsschicht vorhanden ist.

20. Pharmazeutische Zusammensetzung nach einem der Ansprüche 15 bis 19, wobei das Aspirin in dem Kern vorhanden ist und das Betain, das in der Überzugsschicht vorhanden ist, in einer Form mit kontrollierter Freisetzung ist.

21. Pharmazeutische Zusammensetzung nach einem der Ansprüche 15 bis 20, wobei das Betain in dem Kern in einer Form mit kontrollierter Freisetzung vorhanden ist und das Aspirin in der Überzugsschicht vorhanden ist.

22. Pharmazeutische Zusammensetzung nach Anspruch 21, wobei die Überzugsschicht auch ein oder mehrere Puffermittel enthält.

23. Pharmazeutische Zusammensetzung nach Anspruch 22, wobei die Überzugsschicht auch ein oder mehrere Puffermittel und einen oder mehrere Schutzfilm(e) enthält.

24. Pharmazeutische Zusammensetzung nach Anspruch 23, wobei das Betain ausgewählt ist aus der Gruppe bestehend aus Glycin-wasserfreiem Betain und Glycin-Betainmonohydrat.

25. Pharmazeutische Zusammensetzung nach Anspruch 24, des Weiteren enthaltend einen äußeren Schutzüberzug oder eine Abschlussschicht, die die Tablette umgibt.

26. Pharmazeutische Zusammensetzung nach Anspruch 15, wobei das Aspirin die Form von magensaftresistent überzogenen Aspiringranula aufweist.

27. Pharmazeutische Zusammensetzung nach Anspruch 1 in der Form einer zweischichtigen Tablette, die eine erste Schicht umfasst, die Aspiringranula und einen oder mehrere Hilfsstoff(e) umfasst, sowie eine zweite Schicht, die ein Betain und eine oder mehrere Pufferverbindung(en) und einen oder mehrere Hilfsstoff(e) umfasst.

28. Pharmazeutische Zusammensetzung nach Anspruch 27, wobei die erste Schicht Aspiringranula, einen oder mehrere Füllstoff(e) und gegebenenfalls ein Schmiermittel umfasst, und die zweite Schicht ein Betain, gegebenenfalls ein Nassgranulierungsmittel, eine oder mehrere Pufferverbindung(en), ausgewählt aus der Gruppe bestehend aus Kalziumkarbonat, Magnesiumoxid, Magnesiumkarbonat und Gemischen davon, und gegebenenfalls Magnesiumstearat umfasst.

29. Pharmazeutischen Zusammensetzungen nach einem der Ansprüche 15 bis 28, des Weiteren enthalten ein antithrombotisches Mittel oder ein Antikrebsmittel oder ein entzündungshemmendes Mittel oder ein Antibiotikum oder ein Antidiabetikum oder ein Antioxidans.

30. Kombination nach Anspruch 1, wobei die Menge der zweiten Verbindung mindestens die 20-fache, zum Beispiel 30-, 40-, 50-, 70-, 85- oder 100-fache Menge, berechnet als Gewicht der Acetylsalicylsäure, der ersten Verbindung ist.

## Revendications

1. Une combinaison pharmaceutique orale comprenant au moins:
- Un premier composé choisi parmi le groupe consistant en l'acide acétylsalicylique, acide salicylique et leurs sels pharmaceutiques, et
- Un deuxième composé choisi parmi le groupe consistant en la glycine bétaine anhydre et la glycine bétaine monohydrate,
dans laquelle ladite combinaison comprend moins de 100 mg dudit premier composé exprimé en acide acétylsalicylique, et
dans laquelle la quantité du second composé est au moins 5 fois la quantité, calculée en poids d'acide acétylsalicylique, dudit premier composé.

2. La combinaison de la revendication 1, qui comprend une quantité dudit premier composé, calculée en poids d'acide acétylsalicylique, de moins de 85 mg, avantageusement de moins de 75 mg, préférablement de moins de 60 mg.

3. La combinaison de la revendication 1, qui comprend une quantité d'acide acétylsalicylique ou sel pharmaceutique de celui-ci correspondant à 3 à 80 mg, avantageusement de 5 à 75 mg, préférablement de 10 à 75 mg calculés en acide acétylsalicylique.

4. La combinaison de la revendication 1, dans laquelle la quantité du second composé est comprise entre 5 et 25 fois la quantité calculée en poids d'acide acétylsalicylique dudit premier composé.

5. La combinaison de la revendication 1, qui est préparée au moins à partir d'un mélange dans lequel au moins 50% en poids, préférablement au moins 90% en poids du premier composé et au moins 50% en poids, préférablement au moins 90% en poids du second composé sont dans une forme soluble.

6. La combinaison de la revendication 1, dans laquelle le second composé est au moins sous une forme à libération contrôlée et/ou dans laquelle le premier composé est au moins partiellement sous une forme à libération immédiate.

7. La combinaison de la revendication 1, dans laquelle le premier composé est au moins partiellement sous une forme à libération immédiate.

8. La combinaison de la revendication 1, qui comprend des particules sèches, particulièrement des microparticules, préparées en séchant un mélange dans lequel le premier composé et le second composé sont partiellement sous une forme soluble.

9. La combinaison de la revendication 1, dans laquelle le premier composé et le second composé sont combinés dans une forme choisie parmi le groupe consistant en une matrice, un gel, un hydrogel, une cire et un support poreux, une tablette bicouche et leur combinaison.

10. Une forme pharmaceutique unitaire orale comprenant au moins une combinaison pharmaceutique de l'une quelconque des revendications 1 à 9.

11. Un kit pour administration journalière, ledit kit comprenant au moins:
- Une première formulation orale comprenant un premier composé choisi parmi le groupe consistant en l'acide acétylsalicylique, acide salicylique et leurs sels pharmaceutiques, et
- Une deuxième formulation orale comprenant un second composé choisi parmi le groupe consistant en la glycine bétaine anhydre et la glycine bétaine monohydrate,
dans laquelle la première formulation orale comprend moins de 100 mg dudit premier composé exprimé en acide acétylsalicylique, et
dans laquelle la quantité du second composé dans la deuxième formulation orale est au moins 5 fois la quantité, calculée en acide acétylsalicylique, dudit premier composé.

12. Le kit de la revendication 11, dans lequel la première formulation orale comprend une quantité dudit premier composé, calculée en acide acétylsalicylique, de moins de 85 mg, avantageusement de moins de 75 mg, préférablement de moins de 60 mg.

13. Le kit de la revendication 12 qui est adapté pour la préparation d'une combinaison selon l'une quelconque des revendications 2 à 9.

14. L'utilisation d'un
- Premier composé choisi parmi le groupe consistant en l'acide acétylsalicylique, acide salicylique et leurs sels pharmaceutiques, et
- Un deuxième composé choisi parmi le groupe consistant en la glycine bétaine anhydre et la glycine bétaine monohydrate pour la préparation d'un médicament oral selon l'une quelconque des revendications 1 à 9 ou une forme pharmaceutique selon la revendication 10 ou un kit selon l'une quelconque des revendications 11 à 13, pour traiter ou prévenir les perturbations du flux sanguin, ou pour traiter ou prévenir le cancer, ou pour traiter ou prévenir le diabète, ou pour traiter ou prévenir les troubles de l'intestin, ou pour traiter ou prévenir l'inflammation.

15. Une composition pharmaceutique orale qui est une combinaison pharmaceutique selon l'une quelconque des revendications 1 à 9, ladite composition comprenant une bétaine et l'aspirine dans une formulation où la bétaine et l'aspirine sont formulées ensemble dans une tablette bicouche, l'aspirine étant présente dans une première couche, et la bétaine étant présente dans une seconde couche dans une quantité d'au moins cinq fois la quantité d'aspirine.

16. La composition pharmaceutique telle que définie dans la revendication 15, où la couche contenant la bétaine inclut également un ou plusieurs agents tampon.

17. La composition pharmaceutique telle que définie dans la revendication 15, où la tablette inclut un noyau et une couche de revêtement entourant ledit noyau et où l'une des bétaine et aspirine est présente dans le noyau et l'autre est présente dans la couche de revêtement entourant le noyau.

18. La composition pharmaceutique telle que définie dans la revendication 15, où la tablette inclut un noyau et une couche de revêtement entourant ledit noyau et où un mélange de bétaine et d'aspirine est présent dans le noyau et l'une des bétaine et aspirine est présente dans la couche de revêtement entourant le noyau.

19. La composition pharmaceutique telle que définie dans la revendication 17, où l'aspirine est présente dans le noyau et la bétaine est présente dans la couche de revêtement.

20. La composition pharmaceutique telle que définie dans l'une quelconque des revendications 15 à 19, où l'aspirine est présente dans le noyau et la bétaine présente dans la couche de revêtement est dans une forme à libération contrôlée.

21. La composition pharmaceutique telle que définie dans l'une quelconque des revendications 15 à 20, où la bétaine est présente dans le noyau sous une forme à libération contrôlée et l'aspirine est présente dans la couche de revêtement.

22. La composition pharmaceutique telle que définie dans la revendication 21 où la couche de revêtement inclut également un ou plusieurs agents tampon.

23. La composition pharmaceutique telle que définie dans la revendication 22 où la couche de revêtement inclut également un ou plusieurs agents tampon et un ou plusieurs films protecteurs.

24. La composition pharmaceutique telle que définie dans la revendication 23 où la bétaine est choisie parmi le groupe consistant en la glycine bétaine anhydre et la glycine bétaine monohydrate.

25. La composition pharmaceutique telle que définie dans la revendication 24 incluant en plus une couche extérieure de protection ou un revêtement de finition entourant ladite tablette.

26. La composition pharmaceutique telle que définie dans la revendication 15 où l'aspirine est sous la forme de granules d'aspirine à enrobage entérique.

27. La composition pharmaceutique telle que définie dans la revendication 1 sous la forme d'une tablette bicouche qui comprend une première couche comprenant des granules d'aspirine et un ou plusieurs excipients, et une seconde couche comprenant une bétaine et un ou plusieurs agents tampon et un ou plusieurs excipients.

28. La composition pharmaceutique telle que définie dans la revendication 27, où la première couche comprend des granules d'aspirine, un ou plusieurs agents gonflants et optionnellement un lubrifiant, et la seconde couche comprend une bétaine, optionnellement un agent humide de granulation, un ou plusieurs agents tampon choisis parmi le groupe consistant en le carbonate de calcium, l'oxyde de magnésium, le carbonate de magnésium et leurs mélanges, et optionnellement le stéarate de magnésium.

29. La composition pharmaceutique telle que définie dans l'une quelconque des revendications 15 à 28 incluant en plus un agent antithrombotique ou un agent anticancéreux ou un agent anti-inflammatoire ou un agent antibiotique ou un agent antioxydant.

30. La combinaison pharmaceutique de la revendication 1, dans laquelle la quantité du second composé est au moins 20 fois, par exemple 30, 40, 50, 70, 85 ou 100 fois la quantité calculée en poids d'acide acétylsalicylique dudit premier composé.
